# EUROPEAN PATENT APPLICATION

(11) **EP 4 663 200 A1**
(43) Date of publication of application: **17.12.2025**
(21) Application number: 24181172.8
(22) Date of filing: 10.06.2024
(51) Int. Cl.: A61K 39/00, A61K 39/39

(54) **COMPOSITIONS AND METHODS**

(71) Applicant: CPTx GmbH, 82152 Planegg (DE)
(72) Inventor: Sigl, Christian, 82152 Planegg (DE); Honemann, Maximilian N, 82152 Planegg (DE)
(74) Representative: Potter Clarkson

(57) **Abstract**

Provided herein are single strands of DNA that allow for the expression of specific proteins or non-coding RNAs in a host cell or tissue. Also provided herein are vectors, lipid nanoparticles, DNA nanostructures, compositions, and vaccines comprising the single strand of DNA provided herein and uses thereof; and methods of making the same.

## Description

### Field of the invention

The present invention is in the field of therapeutic nucleic acids, more particularly in the field of single-stranded DNA (ssDNA) vaccines and gene therapies.

### Background

Advances in therapeutic nucleic acid technologies have allowed the development of nucleic acid vaccines and gene therapies targeted to a range of genetic and acquired diseases. Established nucleic acid vaccines and gene therapies utilise mRNA or doublestranded DNA (dsDNA), which are typically delivered to a target cell in a vector such as a virus, lipid nanoparticle, or liposome. Translation of the mRNA, or transcription and translation of the dsDNA, produces a polypeptide which may (in the case of a vaccine) be processed and presented as an antigen to a subject's immune system, thereby generating adaptive immunity; or (in the case, for example, of cancer or a genetic disease) may provide a functional heterologous version of an absent or dysfunctional endogenous protein, thereby genetically complementing a pathogenic phenotype. mRNA and dsDNA have been used in vaccination strategies against, for example, COVID-19; and a clinical trial of a non-integrating dsDNA therapy for cystic fibrosis is ongoing.

Despite their promise, however, mRNA and dsDNA vaccines and gene therapies face significant technical challenges. For example, dsDNA vaccines and therapies are often associated with high cellular toxicity, immunogenicity due to interactions with cGAS and STING pathways, and a risk of genomic integrations, contributing a low safety profile. Because of the inherent instability of mRNA, vaccines and gene therapies based on mRNA must be refrigerated until the point of use, which is disadvantageous in countries which lack appropriate cold chains. Furthermore, mRNAs are rapidly degraded in the body following administration, leading to transient expression of antigens and/or therapeutic polypeptides *in vivo.* Transient expression from mRNA can require high levels of mRNA to be delivered a patient to induce the robust adaptive immune response required for vaccination; and, in a gene therapy context, requires frequent re-administration to obtain a therapeutic effect.

There therefore exists a need for improved therapeutic nucleic acids, in particular therapeutic nucleic acid vaccines and gene therapies.

### Brief description of the invention

The invention provides compositions and methods that allow for the use of ssDNA molecules in a wide range of *in vivo* purposes. For example, the single strand of DNA and methods and uses described herein are suitable for use as a vaccine, for example a vaccine against infection with a pathogen or a disease such as cancer. The single strand of DNA and methods and uses described herein are suitable for use in gene therapies, for example by delivering appropriate therapeutic proteins or peptides. The single strand of DNA and methods and uses described herein are also suitable for delivering non-coding RNA to a cell or a subject, for example for RNAi based therapies or CRISPR based gene editing or silencing.

The single strand of DNA and methods described herein are considered to be particularly useful in context where long lengths of DNA are required, for example in the context of encoding large genes, or tandem arrays of genes; and/or in contexts where large quantities of DNA are required, for example in a short time, such as at the onset of a pandemic. Due to the stability of the single stranded DNA relative to mRNA, the present invention is also considered to be particularly suitable for context in which a cold chain is not appropriate or difficult to achieve.

### Detailed description of the invention

The invention provides a range of nucleic acids such as a single strand of DNA, methods, compositions and medical uses, as set out below.

The invention provides a single strand of DNA that comprises a first target sequence operably linked to a first promoter sequence, wherein the first promoter sequence drives expression of the first target sequence to produce a first RNA molecule either:
i) directly from the single strand of DNA; or
ii) following replication of the single strand of DNA into the double strand form.

It will be appreciated that the concept of the invention is a single strand of DNA that ultimately is used as a template for transcription, to produce the first RNA molecule (which as described herein can be a protein coding RNA that can then be translated, or is a non-coding RNA). The skilled person will realise that the sequence of the single strand of DNA can directly be bound by RNA polymerase to instigate transcription (i.e. is the antisense strand); or, the single strand of DNA may itself not comprise the promoter sequence that is recognised by RNA polymerase, and the first target sequence may not itself be transcribed, and may instead require amplification to a double stranded form of the single strand of DNA, where the complementary new strand of DNA comprises the required sequences, i.e. the single strand of DNA maybe the sense or antisense strand with respect to promoter sequences, first target sequences and other sequences, and the disclosure herein should be taken as applying to either a sense or antisense stand. The skilled person will understand therefore that references to particular features, such as a polyA tail are intended to refer to the necessary sequence so as to result in an RNA molecule that comprises a polyA tail, whether the single strand of DNA is sense or antisense with respect to that feature.

Accordingly in one embodiment the single strand of DNA is an antisense strand with respect to the promoter sequence and the first target sequence. In other embodiments the single strand of DNA is the sense strand with respect to the promoter sequence and the first target sequence.

The single strand of DNA of the invention may be designed so as to allow expression of the first target sequence in any cell, for example in a eukaryotic cell or a prokaryotic cell, or in an in vitro transcription or in vitro transcription/translation system. Preferable the single strand of DNA of the invention is designed so as to allow expression in a eukaryotic cell, preferably a mammalian cell, preferably a human cell. The skilled person will recognise the components that are required to ensure expression in a particular context.

In some embodiments, the single strand of DNA is entirely single stranded and/or is substantially single stranded.

In some embodiments, the single strand of DNA contains no regions of self-complementarity. In other embodiments the single strand of DNA contains no regions of self-complementarity of sufficient length so as to allow internal hybridisation or substantial internal hybridisation. For example, in some embodiment the single strand of DNA contains no regions of self-complementarity:
a) that are longer than 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or 100 base pairs; and/or
b) where the combined length of two regions of the single strand of DNA that make up a particular self-complementary region constitute less than 50%, 45%, 40%, 35%, 30%, 25%, 20%, 15%, 10%, 5%, or 2% or less than 1% of the total length of the single strand of DNA.

As will be understood, the single strand of DNA may hybridise to a further nucleic acid strand, for example a further DNA strand and/or further RNA strand.

Although in some embodiments the single strand of DNA is intentionally designed so as to not comprise any or substantially any self-complementarity, in other embodiments the single strand of DNA is designed so as to have intentional regions of self-complementarity, since under some circumstances such internal hybridisation has advantages.

Accordingly, in some embodiments, at least one portion of the single strand of DNA may hybridise to a second portion of the same single strand of DNA. In each case, the single strand of DNA or portion of the single strand of DNA becomes double stranded.

Accordingly, in some embodiments, the single strand of DNA comprises at least one region that is double stranded, wherein said double stranded region is generated via hybridisation between at least a first and second region of the same single strand of DNA that are substantially complementary to each other. In some embodiments the single strand of DNA comprises at least one region that is double stranded, wherein said double stranded region is generated via hybridisation between at least a first and second region of the same single strand of DNA that are substantially complementary to each other and:
a) wherein the hybridisation between a first and second region of the single stand of DNA enhances expression of the first RNA molecule, for example wherein the first region of the single strand of DNA is in the promoter and the second region of the single strand of DNA is distal to the promoter; or
b) wherein the first and second region of the single strand of DNA that are substantially complementary to each other are located at the 5' and the 3' end of the single strand of DNA so that the single strand of DNA is circularised upon hybridisation between the first and second regions.

The skilled person will be aware that certain internal hybridisation events can give rise to the formation of particular secondary structures. Secondary structures in the single strand of DNA (and/or the corresponding RNA product) can impart various properties on the nucleic acid.

Accordingly in some embodiments the single strand of DNA may comprise a secondary structure. Secondary structures are known to the person skilled in the art. In some embodiments, the single strand of DNA comprises a hairpin, for example a hairpin at the 5' and/or 3' end of the single strand of DNA. In some embodiments, the single strand of DNA comprises a secondary structure so as to:
a) increase stability of the single stand of DNA; and/or
b) to increase expression from the promoter.

It may be desirable to direct or restrict expression of a target sequence to a particular cell type. Cell-specific expression of a target sequence may be achieved by selecting a suitable promoter. Accordingly, in some embodiments, the promoter is a promoter capable of driving transcription in a eukaryotic cell, for example in a mammalian cell, for example in a human cell, for example in a dendritic cell, T cell and/or B cell.

In some embodiments, the promoter is a constitutive promoter. In some embodiments, the promoter is an inducible promoter.

The skilled person will understand how to choose a suitable promoter depending on the intended use.

For example, in some embodiments, the promoter is selected from the group comprising or consisting of: Cytomegalovirus (CMV) Immediate-Early promoter; Simian Virus 40 (SV40) promoter; Muscle Creatine Kinase promoter (MCK); or a tissue or cell-type specific promoter, for example dendritic cell, B cell and/or T cell specific promoter. These promoters are considered to be particularly useful in the context of vaccines, for example for the delivery of antigens to dendritic cells, B cells and T cells.

In some embodiments, the promoter is selected from the group comprising or consisting of: Human Elongation Factor 1 Alpha (EF1a) Promoter; Tetracycline-Inducible Promoter; a MiniPromoter; or a tissue or cell-type specific promoter. These promoters are considered to be particularly useful in the context of gene therapy. For example for delivering therapeutic proteins to target cells for example target mammalian cells.

In some embodiments, the promoter is selected from the group comprising or consisting of: U6 promoter; H1 promoter; or a CMV promoter; or a tissue or cell-type specific promoter. These promoters are considered to be particularly useful for the expression of non-coding RNAs, i.e. where the first RNA molecule is a non-coding RNA. The promoter may be a strong promoter or a weak promoter. In some embodiments the promoter is a strong promoter, for example is selected from the group comprising or consisting of the CMV promoter, SP6 promoter or T7 promoter.

In some embodiments, the single strand of DNA further comprises a first enhancer sequence. Enhancers are well known to the skilled person and aid in increasing expression of the first target sequence from the promoter. Enhancers can be cell-type specific. Tor example in some embodiments the enhancer enhances expression of the first target sequence in a specific cell type. Enhancers can act as an enhancer when in the single strand of DNA itself, or can act as an enhancer once transcribed in to RNA. Enhancers can be located in the 5'UTR, 3'UTR or both 5' and 3' UTR. Accordingly in one embodiment the single strand of DNA or the first RNA molecule comprises at least one enhancer sequence is located in the 5'UTR, 3'UTR or in the 5'UTR and 3'UTR.

The enhancer may be any enhancer. Examples of suitable enhancers include:
a) viral enhancers, for example the 72-bp enhancer element from Simian Virus 40 (SV40), the enhancer from CMV, and/or the enhancer from Rous sarcoma virus;
b) Ubiquitous/Constitutive Enhancers, for example the enhancer from the human elongation factor 1 alpha (EF1a) enhancer;
c) tissue/Cell-Type Specific Enhancers, for example the muscle creatine kinase (MCK) enhancer for muscle cells;
d) Super-enhancers associated with genes defining cell identity;
e) Synthetic/Designed Enhancers; and/or
f) inducible enhancers, for example an inducible enhancer that response to hormones or small molecules.

In some embodiments the single strand of DNA or first RNA molecule comprises one enhancer. In other embodiments the single strand of DNA or first RNA molecule comprises at least two or more enhancers. For example in some embodiments the single strand of DNA or first RNA molecule comprises at least two enhancers of any of (a)-(f) above, for example a combination of the SV40 enhancer with a muscle-specific enhancer for muscle targeting.

The single strand of DNA of the invention has many uses, including therapeutic uses. To achieve these uses the single strand of DNA has to be delivered to a particular cell. Once inside the cell the single strand of DNA is transported to the nucleus where it is replicated to a double strand form. The promoter then drives transcription, and where the first target sequence is protein coding sequence, the transcript is translated along with other mRNAs in the cell.

To aid in this process, in some embodiments the single strand of DNA comprises a nuclear localisation signal. In other embodiments the single strand of DNA comprises inverted-terminal repeat like hairpin motifs. It will be appreciated that disclosures herein can be combined, so that for example in some embodiments the single strand of DNA comprises substantially no internal complementarity but does comprise inverted-terminal repeat like hairpin motifs to aid in expression.

Similarly, the single strand of DNA may in some embodiments be linear, but comprises caps formed by hairpin loops at the 5' end and/or the 3' end.

In some embodiments the single strand of DNA is linear and does not comprise any hair pin loops.

In some embodiments the single strand of DNA is circular. There are various means for producing circular single stranded DNA. For example in some embodiments the single strand of DNA is circularised via complementary base pairing of the 5' end and the 3' end of the single strand of DNA. Accordingly in some embodiments the single strand of DNA has a 5' and 3' terminal sequence that are complementary to one another or substantially complementary to one another sufficient to allow hybridisation.

In some embodiments the single strand of DNA is linear, and is capable of circularising when in a cellular environment, for example a eukaryotic cell, for example a mammalian cell, for example a human cell; and or is capable of circularising in an in vitro transcription translation system, for example in a mammalian in vitro transcription translation system.

Since in some preferred embodiments the single strand of DNA is for expression in a cell, in some instances the single strand of DNA comprises modifications that aid in expression, or for example render the nucleic acid more stable in the cellular context, or that help to make the single strand of DNA less immunogenic. For example in some embodiments the single strand of DNA is modified in such a way so as to enhance expression in a mammalian cell, for example a human cell.

In some embodiments the modification is to reduce immunogenicity, for example reduce immunogenicity in a mammalian cell. In some instances the modification is methylation.

In some embodiments the modification is to make the single strand of DNA act as an adjuvant. For example non-methylated DNA is able to act as an adjuvant, so in some embodiments the modification is de-methylation.

It is considered to be advantageous in some situations to enable to expression of multiple proteins or peptides, and/or multiple non-coding RNAs within the same cell or tissue. An advantage of the present invention is that it is possible to produce large amounts of single strand DNA, and large amounts of long read single strand DNA (see for example WO 2018054571). The ability to produce long strands of single strand DNA opens up the possibility of encoding multiple genes/non-coding RNAs on the same physical single strand DNA molecule; and/or encoding large proteins or large non-coding RNA for delivery into a subject for example for therapeutic purposes.

Accordingly in one embodiment the single strand of DNA of the invention additionally comprises a second target sequence, or a third, fourth, fifth, sixth, seventh, eighth, ninth or tenth or more target sequence. The single strand of DNA may comprise any number of target sequences. The target sequences may all be different, or at least two or more of the target sequences may be the same. For example in some embodiments: each target sequence is the same as the first target sequence; or the single strand of DNA comprises at least two target sequences that are different to each other.

The target sequences may be present in the single strand of DNA in any order or orientation.

In some embodiments at least two of the target sequences are operably linked to different promoters.

Preferably the multiple target sequences that present in the single strand of DNA are arranged in a tandem array. In preferred embodiments all of the target sequences present in the single strand of DNA are arranged in the same orientation so that transcription from a single promoter located at one end of the DNA (for example the 5' end) is able to drive transcription through all of the target sequences from a single promoter to produce a single transcript.

Accordingly in some embodiments all of the target sequences present in single strand of DNA in a tandem array. In some embodiments all of the target sequences present in single strand of DNA in a tandem array are operably linked to the same promoter. In preferred embodiments then where the single strand of DNA comprises multiple target sequences, the target sequences are transcribed into a single RNA molecule.

It will be appreciated that in these scenarios, to allow initiation of translation from each of the target sequences (in scenarios where the target sequence is a protein coding sequence), the RNA molecule should comprise an internal ribosome entry site (IRES) that is present 5' to each target sequence (each protein coding target sequence), so that each target sequence is translated, for example wherein the IRES is an IRES from encephalomyocarditis virus (EMCV) or poliovirus. Since the RNA molecule is transcribed from the single strand of DNA of the invention, the single strand of DNA of the invention also comprises sequences that when transcribed into RNA are capable of acting as an IRES.

In addition to IRES, where any one or more target sequences is a protein coding sequence that requires translation, the skilled person will understand that appropriate start and stop codon are also necessary.

In some instances, spacer sequences are present between the IRES and one or more target sequences.

Regardless of how many target sequences are present in the single strand of DNA of the invention, proper transcription requires a transcription terminator.

Of course, the single strand of DNA of the invention may comprise multiple promoter/target sequence units. For example may comprise a first promoter associated with a first set of target sequences (that may be one or more target sequences) target sequence and a second promoter associated with a second set of target sequences (that may be one or more target sequences). Multiple promoters can then be used to achieve differential effects. For example one promoter may be a constitutive promoter whilst a second promoter may be an inducible promoter. The promoters may interact via expressed inducers or repressors to build up complex genetic circuits using logic gates.

As set out elsewhere, the single strand of DNA of the invention may correspond to the sense or antisense strand of a particular sequence. For example in some instances, RNA polymerase is able to directly associate with the single strand of DNA of the invention and initiate transcription. In other cases RNA polymerase is not able to associate directly with the single strand of DNA of the invention, but is able to associate with the corresponding complementary strand which will be produced following replication of the single strand of DNA, for example replication in the nucleus of a eukaryotic cell. The skilled person will know how to design the various component parts depending on which strand is being employed.

As set out above, an advantage of the present invention is the ability to produce and package large amounts of nucleic acid sequence that can code for large proteins, large non-coding RNAs, multiple copies of the same protein/non-coding RNA and/or multiple copies of different proteins/non-coding RNAs.

Accordingly in some embodiments the single strand of DNA is:
At least 500 nucleotides, or at least 1000 nucleotides or at least 1250, 1500, 1750, 2000, 2250, 2500, 2750, 3000, 3250, 3500, 3750, 4000, 4250, 4500, 4750, 5000, 5250, 5500, 5750, 6000, 6250, 6500, 6750, 7000, 7250, 7500, 7750, 8000, 8250, 8500, 8750, 9000, 9250, 9500, 9750, 10000, 10250, 10500, 10750, 11000, 11250, 11500, 11750, 12000, 12250, 12500, 12750, 13000, 13250, 13500, 13750, 14000, 14250, 14500, 14750, 15000, 15250, 15500, 15750, 16000, 16250, 16500, 16750, 17000, 17250, 17500, 17750, 18000, 18250, 18500, 18750, 19000, 19250, 19500, 19750, or at least 20000 nucleotides in length; and/or
Between 1000 and 20000, 1250 and 19750, 1500 and 19500, 1750 and 19250, 2000 and 19000, 2250 and 18750, 2500 and 18500, 2750 and 18250, 3000 and 18000, 3250 and 17750, 3500 and 17500, 3750 and 17250, 4000 and 17000, 4250 and 16750, 4500 and 16500, 4750 and 16250, 5000 and 16000, 5250 and 15750, 5500 and 15500, 5750 and 15250, 6000 and 15000, 6250 and 14750, 6500 and 14500, 6750 and 14250, 7000 and 14000, 7250 and 13750, 7500 and 13500, 7750 and 13250, 8000 and 13000, 8250 and 12750, 8500 and 12500, 8750 and 12250, 9000 and 12000, 9250 and 11750, 9500 and 11500, 9750 and 11250, 10000 and 11000, 10250 and 10750 nucleotides in length.

In some preferred embodiments the single strand of DNA is: at least 5000 nucleotides in length. In other preferred embodiments the single strand of DNA is at least 7000 nucleotides in length.

The single strand of DNA of the invention has therapeutic uses, since it can be used to deliver antigens or therapeutic proteins (for example) to a subject or a cell in need thereof. Accordingly in preferred embodiments the single strand of DNA is designed so as to allow expression of the first target sequence to produce a first RNA molecule in eukaryotes, for example in mammals, for example in humans. As set out above, promoters, terminators etc can be chosen by the skilled person to allow transcription and downstream translation (where required) in a eukaryote for example a mammal, for example a human.

In some embodiments the single strand of DNA is used to produce a protein or a peptide. As set out above the single strand of DNA may comprise multiple target sequences and some in some embodiments where the single strand of DNA does comprise multiple target sequences, at least one of the target sequences may be a sequence that encodes a protein or peptide.

As set out above, the single strand of DNA may be sense or antisense with respect to any transcript that is intended to be produced. Accordingly in some embodiments the first target sequence is:
a) an antisense strand that encodes a first protein or peptide and is able to be directly transcribed to produce the first RNA molecule; or
b) a sense strand that is able to be transcribed to produce the first RNA molecule once the single strand of DNA is replicated to produce the antisense strand.

Reference to the first target sequence being a protein or peptide encoding sequence is intended to capture both the sense and antisense embodiments that result in the production of said protein or peptide.

Where the first target sequence is a protein or peptide encoding sequence, the first target sequence comprises one or more sequences that when transcribed into the first RNA molecule form a 5'UTR, a 3'UTR and/or a polyA tail.

The 5'UTR and/or 3'UTR may comprise sequences that form RNA secondary structures that regulate gene expression in eukaryotes, for example wherein:
a) the 5'UTR comprises:
   i) one or more stable hairpin and/or stem-loop structures to downregulate gene expression; and/or
   ii) an internal ribosome entry site (IRES), for example wherein the IRES is an IRES from encephalomyocarditis virus (EMCV) or poliovirus;
      and/or
b) the 3'UTR comprises:
   i) one or more stem-loop structures and/or AU rich elements; and/or
   ii) one or more miRNA binding sites.

In some embodiments the first protein or peptide is an antigenic protein or peptide. In some embodiments the first protein or peptide comprises an antigenic protein or peptide, for example the protein or peptide itself may not be antigenic until processed by a cell to produce an antigenic fragment.

Antigenic proteins or peptides are presented (at least in mammals such as humans) to the immune system via complex formation with proteins of the major histocompatibility proteins (MHC). Major histocompatibility complex (MHC) proteins are crucial components of the immune system that play a key role in recognizing foreign molecules and initiating immune responses. MHC proteins are glycoproteins found on the cell surface. They are categorized into two main classes: MHC Class I: Found on the surface of almost all nucleated cells. MHC Class 1 presents peptides from within the cell)to cytotoxic T cells (CD8+ T cells). This is essential for the immune system to recognize and destroy infected or cancerous cells; MHC Class II: Primarily expressed on antigen-presenting cells (APCs) such as dendritic cells, macrophages, and B cells. MHC Class II presents peptides from outside the cell to helper T cells (CD4+ T cells). This is crucial for the activation of T cells and the coordination of the immune response.

Different processing routes can direct a particular protein or peptide for presentation via MHC I or MHC II, for example ubiquitination tends to direct the presentation via MHC I, whereas targeting to the lysosome directs presentation via MHC II.

In some embodiments then the first protein or peptide that is an antigenic protein or peptide or comprises an antigen protein or peptide comprises one or more sequences to enhance presentation of the antigen protein or peptide or fragments thereof, for example:
a) presentation by the major histocompatibility proteins (MHC), for example MHC I and/or MHC II; and/or
b) presentation by antigen presenting cells, for example dendritic cells, macrophages and/or B cells.

The one or more sequences to enhance presentation of the first protein or peptide fragment may be:
a) a ubiquitin sequence;
b) an Endoplasmic Reticulum (ER) signal peptide, for example the signal peptide from tissue plasminogen activator (tPA;
c) the Lysosome-Associated Membrane Protein 1 (LAMP-1); and/or
d) a chemokine ligand, for example XCL1.

The ubiquitin sequence and the ER signal peptide direct the protein or peptide for presentation via MHC I. Fusion of the antigen with ubiquitin can target it for degradation by the proteasome, enhancing the generation of peptide fragments for presentation via MHC class I molecules.

The LAMP-1 protein can be fused to the target antigenic protein or peptide to direct them to the lysosomal pathway, enhancing presentation via MHC class II molecules and stimulating helper T cell responses.

Fusion of antigens with chemokines like XCL1 can target the antigen to specific dendritic cell subsets, enhancing antigen presentation and immune response.

In some embodiments the first protein or peptide is a protein or peptide present in a pathogen, for example wherein the pathogen is a bacterial pathogen, viral pathogen, fungal pathogen or a protist pathogen. Such embodiments have clear uses in the context of vaccines to prevent or treat infection with a pathogen, as described herein. The protein or peptide present in a pathogen may be any protein or peptide present in a pathogen. Preferably the protein or peptide present in a pathogen is an antigen protein or peptide, or is a protein or peptide that comprises an antigen protein or peptide fragment, for example following intracellular processing.

In some embodiments the first protein or peptide is a disease-causing or disease-associated protein or peptide, such as a cancer antigen or a cancer neoantigen, or an autoimmune antigen or a neurological antigen, or is a protein or peptide that comprises a cancer antigen or cancer neoantigen, or comprises an autoimmune antigen or comprises a neurological antigen for example following intracellular processing. Such embodiments have clear uses in the context of vaccines to prevent or treat diseases, such as treat or prevent cancer or metastasis, treat or prevent autoimmune diseases or treat or prevent neurological diseases, as described herein. The protein or peptide present that is a cancer antigen or cancer neoantigen may be any protein or peptide that is a cancer antigen or cancer neoantigen.

In the context of proteins or peptides from a pathogen, the protein or peptide may be:
a) a viral antigen, for example
   i) an influenza antigen, for example Influenza Hemagglutinin, for example from H5Nx strains;
   ii) a coronavirus antigen, for example from SARS-CoV-2, SARS-CoV, HCoV NL63, HCoV HKU1, or MERS-CoV; for example a spike protein, for example the spike protein from any one or more of from SARS-CoV-2, SARS-CoV, HCoV NL63, HCoV HKU1, or MERS-CoV;
   iii) a HIV antigen, for example HIV-1 ENV or HIV-1 GAG; or
   iv) a Hepatitis B antigen, for example Hepatitis B Surface Antigen (HBsAg);
b) a bacterial antigen, for example
   i) an antigen from Mycobacterium tuberculosis, for example Ag85 Complex, for example from any of Ag85A, Ag85B, Ag85C); ESAT-6; CFP-10
   ii) an antigen from Bacillus anthracis, for example Protective Antigen (PA);
   iii) an antigen from Clostridium tetani, for example tetanus toxin (TeNT)
   iv) an antigen from Escherichia coli, for example Colonization Factor Antigen I (CFA/I);
   v) Salmonella enterica, for example Flagellin (FliC), Invasin Proteins (e.g., SipA, SipC);
   vi) Streptococcus pneumoniae, for example Pneumolysin (Ply), PspA (Pneumococcal Surface Protein A);
   vii) Helicobacter pylori, for example Urease Subunits (UreA, UreB);
   viii) Listeria monocytogenes, for example Listeriolysin O (LLO);
   ix) Shigella spp., for example Ipa Proteins (e.g., IpaB, IpaC);
   x) Vibrio cholerae, for example Cholera Toxin (CT), for example the B subunit of cholera toxin (CTB);
c) a fungal antigen, for example
   i) Candida albicans, for example Agglutinin-like sequence 3 (Als3), Secreted aspartyl proteinases (Sap), Heat shock protein 90 (Hsp90), Hyphal wall protein 1 (Hwp1), or Enolase (Enol);
   ii) Aspergillus fumigatus, for example Aspf3 (Allergen), Catalase, Gel1 (1,3-β-glucanosyltransferase), or Pmp20 (Putative Plasma Membrane Protein);
   iii) Cryptococcus neoformans, for example Glucosylceramide (GlcCer), Mannoproteins (MP98, MP88), Phospholipase B1 (Plb1), or Capsular polysaccharide (GXM);
   iv) Coccidioides posadasii/immitis, for example Antigen 2/PRA (Proline-rich antigen), Chitinase, Urease, Fba (Fructose-bisphosphate aldolase),
   v) Histoplasma capsulatum, for example Histone 2B (H2B), Heat shock protein 60 (Hsp60), Catalase B (CatB), Yps3 (Yeast protein 3);
   vi) Paracoccidioides brasiliensis, for example Glycoprotein 43 (Gp43) peptide 10 (P10), Paracoccin, Triosephosphate isomerase (Tpi),
   vii) Pneumocystis jirovecii, for example Major surface glycoprotein (Msg), Kexin, Monosaccharide transporter (Mst);
      and/or
g) a protist antigen.

The single strand of DNA of the invention may comprise multiple target sequences each encoding different antigens from different strains of the same pathogen species, for example the single strand of DNA of the invention may allow for the expression or 1, 2, 3, 4, 5, 6, 7, 8, 9, 10 or more different antigens from different influenza strains.

In the context of a first protein or peptide that is a disease-causing or disease-associated protein or peptide, such as a cancer antigen or a cancer neoantigen, or an autoimmune antigen or a neurological antigen, or is a protein or peptide that comprises a cancer antigen or cancer neoantigen, or comprises an autoimmune antigen or comprises a neurological antigen:
the cancer antigen may be NY-ESO-1, MAGE-A3, HER2/neu; or PSA;
the Autoimmune Disease Antigen may be insulin, Myelin Basic Protein (MBP); and/or
the Neurological Disease Antigen may be Amyloid Beta (Aβ) or Tau Protein.

A mutant form of ubiquitin (e.g., G76V) can be used to prevent deubiquitination and stabilize the fusion protein.

In addition to an antigenic component, vaccine formulations also often comprise an adjuvant. Adjuvants are essential components of many vaccines, enhancing the immune response to the antigen, modulating the type of immunity generated, and allowing for lower doses of antigens to be used effectively. Some adjuvants are inorganic, such as alum adjuvants, such as aluminum hydroxide or aluminum phosphate. However some adjuvants are of a type that can be expressed by a cell, for example are protein-based or nucleic acid based. In some embodiments therefore it is possible for the single strand of DNA of the invention to encode one or more adjuvants as set out here. Accordingly in some embodiments one or more of the target sequences of the single strand of DNA of the invention encodes a protein or nucleic acid based adjuvant. In preferred embodiments the single strand of DNA encodes both one or more antigenic proteins or peptides, or proteins or peptides that comprise an antigenic portion, and also encode one or more protein or nucleic acid based adjuvants.

The adjuvant may be any protein or nucleic acid based adjuvant. Exemplary adjuvants that are considered to be suitable for use in the invention are:
a) Bacterial protein adjuvants, for example FomA porin from Fusobacterium; MOMP from Shigella flexneri, Porin from Shigella dystenteriae; OmpU from Vibrio cholerae; PorB from Neisseria meningitidis; OmpC and OmpF from Samlonella thyphi, ESAT-6 from Mycobaceterium tuberculosis; rBCSP31 from Brucella abortus; DnaJ-ΔA146Ply from Steptococcus spneumonia; Endopeptidase O from Steptococcus pneumoinae; Cholera toxin from Vibrio cholerae; B-pentamers of LT-IIa and LT-IIb from E. coli; Omp16 from Brucella abortus; BLS from Brucella spp. GrpE from M. tuberculosis; RpfE from M. tuberculosis; Rv0652 from from M. tuberculosis; HBHA from from M. tuberculosis; P97 protein from Mycoplasma hyopneumoniae; Flagellin from Salmonella species; Entolimod (CBLB502) optimised from Salmonella flagellin;
b) One or more cytokines, for example wherein the cytokine is selected from the group comprising or consisting of Interleukin-12 (IL-12), Interleukin-2 (IL-2), Granulocyte-Macrophage Colony-Stimulating Factor (GM-CSF);
c) one or more cytokines, for example wherein the chemokine is CCL21 (Secondary Lymphoid Tissue Chemokine, SLC);
d) one or more co-stimulatory molecules, for example wherein the co-stimulatory molecule is CD40 Ligand (CD40L), or B7-1 (CD80) and B7-2 (CD86);
e) one or more Toll-Like Receptor (TLR) Ligands, for example wherein the Toll-Like Receptor (TLR) Ligand is CpG Oligodeoxynucleotides (CpG ODNs)
f) Flt3 Ligand (Flt3L); and/or
g) Heat Shock Proteins (HSPs)
or any combinations thereof.

As set out above, the single strand of DNA of the invention is considered to be useful in the context of vaccination against infections or diseases such as cancer, autoimmune disease and neurological diseases.

However in the same or different embodiments, the first target sequence, or at least one of the target sequences encodes a non-antigenic protein or peptide. For instance in some embodiments the first target sequence or at least one of the target sequences encodes a therapeutic protein or peptide.

In some embodiments the therapeutic protein or peptide is capable of replacing a defective or deficient protein associated with a genetic disorder. It will be clear than that the single strand of DNA of the invention can be used in gene therapy.

The genetic disorder can be any genetic disorder where expression of a particular protein of non-coding RNA can exert a therapeutic effect. In some particular embodiments the genetic disorder is selected from the group comprising or consisting of: adenosine deaminase severe combined immunodeficiency (ADA-SCID), neuronal ceroid lipofuscinosis, aspartylglucosaminuria, Batten disease, beta-thalassemia, chronic granulomatous disease (CGD), cystic fibrosis, cystinosis, Duchenne muscular dystrophy, Fabry disease, Gaucher disease type I, Gaucher disease type II, Gaucher disease type III, Huntingdon's disease, Hurler disease, Leber's congenital amaurosis, Maroteau-Lamy disease, metachromatic leukodystrophy, Morquio disease type A, Morquio disease type B, mucolipidosis type I, mucolipidosis type II, mucolipidosis type III, mucolipidosis type IV, Niemann-Pick disease type A, Niemann-Pick disease type B, Niemann-Pick disease type C1, Niemann-Pick disease type C2, Pompe disease, retinitis pigmentosa, Sandhoff disease, sickle cell disease, Spinal Muscular Atrophy, X-ALD, hemophilia A, hemophilia B, Hunter disease (MPS II), lysosomal storage disorder, Maroteaux-Lamy disease (MPS VI), Neuronal ceroid lipofuscinosis (NCL) including CLN1 disease, Sanfilippo disease type A (MPS IIIA), Sanfilippo disease type B (MPS IIIB), Sanfilippo disease type C (MPS IIIC), Sanfilippo disease type D (MPS IIID), Schindler disease type I, Schindler disease type II, Sly disease (MPS VII), Tay-Sachs disease.

As mentioned elsewhere herein, the first target sequence that is present in the single strand of DNA of the invention can in some instances be transcribed and translated in to a protein. However in other instances the first target sequence may be a sequence that encodes a non-coding RNA.

Accordingly in some embodiments the first target sequence is a non-coding RNA, for example a functional RNA, for example selected from the group comprising or consisting of:
a) a gRNA
b) an RNA designed to have sequence complementarity to a host target RNA so as to cause RNAi when expressed in a host cell, for example an miRNA; and/or
c) a IncRNA.

As will be apparent to the skilled person, in some instances the first target sequence is designed to that when transcribed the RNA has a sequence that is complementary or substantially complementary to a host target RNA sequence (as mentioned elsewhere the skilled person will appreciate that the single strand of DNA may be designed so that the transcript produced from the single strand of DNA is itself complementary to the target; or, since the single strand of DNA may be sense or antisense, the single strand of DNA may designed so that only following replication to the double stranded form, is transcription initiated, so generating the RNA with a sequence that is complementary to the target).

In some embodiments the single strand of DNA is designed so that the RNA transcript has sequence complementarity to a host target RNA that is RNA transcribed from an oncogene, for example wherein the oncogene is selected from the group comprising or consisting of KRAS, EGFR (Epidermal Growth Factor Receptor), CFTR (Cystic Fibrosis Transmembrane Conductance Regulator), DMD (Dystriophin).

In some instances the single strand of DNA comprises at least a first target sequence that encodes a protein or peptide, and at least a second target sequence that is not a protein or peptide coding sequence and wherein the RNA is a functional RNA. The protein or peptide may be any protein or peptide and the RNA may be any functional RNA.

Particular uses of this embodiment relate to the use of CRISPR gene editing or gene regulation. For example in some embodiments the protein or peptide is a Cas protein or peptide, or equivalent thereof, and the RNA is a gRNA. In some instances the gRNA is designed to have sequence complementarity to a viral genome or viral nucleic acid, for example wherein the virus is selected from the group comprising or consisting of HBV for example wherein the viral nucleic acid is cccDNA (Covalently Closed Circular DNA), or HIV Provirus.

As indicated above, the single strand of DNA of the invention can comprise multiple target sequences. Each of these target sequences can encode any number and any combination of proteins or peptides, and/or non-coding RNAs.

For example in some embodiments the single strand of DNA comprises a second target sequence, or a third, fourth, fifth, sixth, sevenths, eighth, ninth or tenth or more target sequences and wherein each target sequence encodes, or encodes following replication of the single strand of DNA , any one or more of:
a) a protein or peptide that is an antigenic protein or peptide or comprises an antigenic protein or peptide;
b) a protein or peptide that is a therapeutic protein or peptide;
c) an adjuvant protein or peptide; and/or
d) a non-coding RNA, for example a functional RNA, for example selected from the group comprising or consisting of: i) a gRNA, ii) an RNA designed to have sequence complementarity to a host target RNA so as to cause RNAi when expressed in a host cell; and/or iii) a IncRNA.

In some embodiments the single strand of DNA comprises 2, 3, 4, 5, 6, 7, 8, 9, 10 or more target sequences that each encodes, or encodes following replication of the single strand of DNA , an antigenic protein or peptide.

In some embodiments at least two of the target sequences encode, or encodes following replication of the single strand of DNA, different antigenic proteins or peptides, for example at least 3, 4, 5, 6, 7, 8, 9 or at least 10 of the target sequences encode, or encode following replication of the single strand of DNA , different antigenic proteins or peptides.

In the same or different embodiments, at least two of the target sequence encode, or encode following replication of the single strand of DNA , the same antigenic protein or peptide, for example wherein at least 3, 4, 5, 6, 7, 8, 9 or at least 10 of the target sequences encode, or encode following replication of the single strand of DNA , the same antigenic proteins or peptides.

It will be appreciated that there are some instances where when the single strand of DNA encodes more than one antigenic protein or peptide, it may be beneficial to direct the antigenic protein or peptide towards different MHC presentation pathways. Accordingly in some embodiments at least two of the target sequences encode, or encode following replication of the single strand of DNA, an antigenic protein or peptide that each comprise one or more sequences to enhance presentation of the protein or peptide or fragments thereof,
and wherein at least two of the antigenic proteins or peptides comprise different sequences to enhance presentation of the protein or peptide or fragments thereof, for example wherein the sequences to enhance presentation of the protein or peptide or fragments thereof are selected from the group comprising or consisting of:
a) a ubiquitin sequence;
b) an Endoplasmic Reticulum (ER) signal peptide, for example the signal peptide from tissue plasminogen activator (tPA;
c) the Lysosome-Associated Membrane Protein 1 (LAMP-1); and/or
d) a chemokine ligand, for example XCL1.

In some embodiments at least two of the target sequences encode, or encode following replication of the single strand of DNA, the same antigenic protein or peptide and each protein or peptide comprises one or more sequences to enhance presentation of the first protein or peptide or fragments thereof, but wherein the one or more sequences to enhance presentation of the first protein or peptide or fragments thereof present in the first target sequence are different to those of the second target sequence.

The single strand of DNA of the invention may take any physical form. For example in some embodiments the single strand of DNA may be linear or circular.

In some embodiments the single strand of DNA of the invention is not a closed linear DNA. In some embodiments the single strand of DNA of the invention is not dbDNA. In some embodiments the single strand of DNA of the invention does not comprise a telomerase target sequence or a protelomerase target sequence. In some embodiments the single strand of DNA of the invention does not comprise a hairpin loop at the 5', 3' or 5' and 3' termini.

The single strand of DNA of the invention may be made by any means. One particularly useful method of producing the single strand of DNA is as described in WO 2018/054571. Such a method is able to produce very large quantities of single stranded DNA in a very short time. Such an approach is critical for scalable production of vaccines, and in particular is critical in cases such as pandemics where large amounts of new vaccines are required in a very short time period. Current methods of producing alternative vaccines, such as those that utilise mRNA, are not able to achieve the scale and speed of production as are available for the production of vaccines comprising single strands of DNA. The combination of this method of production set out in WO 2018/054571, and methods of production set out herein, and vaccines comprising single strands of DNA is considered to be particularly powerful.

For example in some embodiments the single strand of DNA of the invention is produced by expressing ssDNA from a dsDNA template vector that comprises at least one, preferably at least two, copy of the single strand of DNA of the invention, wherein the one or each copy of the single strand of DNA is flanked by a self-cleaving DNA sequence, to produce a precursor template ssDNA. Accordingly the invention provides a dsDNA template vector that comprises at least one, preferably at least two, copies of the sequence corresponding to the single strand DNA of the invention, wherein the dsDNA vector comprises self-cleaving DNA sequences either side of each copy of the sequence corresponding to the single strand DNA of the invention.

In preferred embodiments expression from the dsDNA template vector occurs in a microbial cell culture. Accordingly the invention also provides a cell, for example a microbial cell, that comprises the dsDNA template vector. The microbial cell may be any microbial cell. Exemplary microbial cells may be selected from the group comprising or consisting of:
a) a bacterial cell for example an E. coli cell, for example a K12-derived E. coli safety strain, for example DH5alpha, XL-1blue or JM109; or
b) a yeast cell.

In preferred embodiments the dsDNA template vector is a phagemid, for example further comprising:
a) a packaging sequence;
b) component(s) ensuring propagation of the phagemid during cell division; and/or
c) a selection marker, typically an antibiotic resistance gene.

Following expression of precursor template ssDNA from the dsDNA template, the method may further comprise isolating the single strand of DNA from the microbial cell or cell culture media. Following isolation, where the precursor template ssDNA comprises self-cleaving DNA sequences, the precursor template ssDNA is digested under reaction conditions where the self-cleaving DNA sequences become active to produce the single strand of DNA, for example wherein said conditions comprise the addition of Zn2+.

The self-cleaving DNA sequences may be self-cleaving desoxyribozymes or DNAzymes, for example Zn 2+ -dependent DNAzymes, for example I-R3.

In some embodiments the dsDNA template vector comprises two, three, four or more, sequences that correspond to the single strand of DNA of the invention, for example wherein each of the sequences is separated with a self-cleaving DNA sequence as set out herein.

It will be clear to the skilled person that where the dsDNA template vector is a phagemid, additional components are required for appropriate assembly and release of the DNA containing phage-like particles. In view of this, in some embodiments, expression of the template precursor ssDNA from the dsDNA template vector occurs in the presence of a helper plasmid or a helper phage which comprises:
a) genes encoding the proteins of a bacteriophage, e.g. M13 bacteriophage;
b) component(s) ensuring propagation of the helper plasmid during cell division; and/or
c) a selection marker, for example an antibiotic resistance gene.

In preferred embodiments, of producing the single strand of DNA of the invention, the microbial cell is a bacterial cell and the single strand of DNA is packaged into phage-like particles which are secreted from the bacterial cells, and wherein the phage-like particles are isolated from the cell culture and the phagemid ssDNA is purified from the phage-like particles.

It will be clear to the skilled person that where the single strand of DNA of the invention is made according to the method set out above, that utilises self-cleaving DNA sequences, that following cleavage of those sequences, the 5' and 3' end of the single strand of DNA will have fixed end sequences, i.e. sequences that correspond to the scars remaining from cleavage of the self-cleavage DNA sequence. Accordingly in some embodiments the single strand of DNA of the invention comprises 5' and 3' end sequences are the scars that remain following DNAzyme cleavage.

An advantage of the present system over the delivery of proteins using mRNA is that single stranded DNA is inherently more stable that mRNA. DNA contains the sugar deoxyribose, while RNA contains ribose. The absence of a 2'-hydroxyl group in DNA's deoxyribose makes it chemically more stable than RNA. RNA also undergoes spontaneous hydrolytic cleavage approximately 100 times faster than DNA. This increased susceptibility in RNA is attributed to the intramolecular attack of the 2'-hydroxyl function on the neighbouring phosphate diester, leading to a 2',3'-cyclic phosphate. DNA's lack of a 2'-OH group makes it less prone to such cleavage, enhancing its stability over an organism's lifetime. Furthermore, the DNA duplex is more hydrated compared to the RNA duplex. This difference in hydration levels is speculated to influence their stability, with the more stable RNA duplex having the lowest hydration level. Although specific hydration numbers are not provided, the general trend indicates that lower hydration correlates with higher stability in single strand of DNA duplexes.

High temperatures increase the rate of spontaneous and irreversible RNA backbone hydrolysis, a phenomenon not typically observed for DNA.

In some embodiments the single strand of DNA of the invention is stable at a temperature of at least 18°C, or at least 19°C, 20°C, 21°C, 22°C, 23°C, 24°C, 25°C, or 26°C, for example stable for at least 24 hours, 48 hours, 1 week, 2 weeks, 4 weeks, 2 months, 6 months, 1 year or more.

As set out above, the invention also provides a dsDNA template vector from which the single strand of DNA of the invention may be produced. In some embodiments the vector comprises multiple repeats of the sequence corresponding to the single strand of DNA of the invention, for example wherein the multiple repeats are arranged in a head-to-tail orientation, for example wherein the dsDNA vector comprises at least 2, 3, 4, 5, 6, 7, 8, 9, 10, 11, 12, 13, 14, 15, 16, 17, 18, 19, 20, 25, 30, 35, 40, 45, 50 or more repeats.

In some instances the dsDNA vector of the invention is a phagemid. In some instances the phagemid further comprises: a) a packaging sequence; b) component(s) ensuring propagation of the phagemid during cell division; and/or c) a selection marker, typically an antibiotic resistance gene.

The phagemid may further comprise: a) genes encoding the proteins or parts of a protein of a bacteriophage, for example proteins or parts of a protein of the M13 bacteriophage; b) component(s) ensuring propagation of the helper plasmid during cell division; and/or c) a selection marker, for example an antibiotic resistance gene.

It will be appreciated that since there are clear therapeutic uses of the single strand of DNA of the invention, the single strand of DNA the invention must be administered to a subject in need thereof, for example administered to a mammal, for example to a human. Accordingly the invention provides various formulations of the single strand of DNA of the invention that are suitable for delivery of the single strand of DNA to a subject, or to a specific tissue or site in the subject. Preferences for features of the formulations are as defined elsewhere herein.

In some embodiments the formulation is a lipid nanoparticle formulation. The invention therefore provides a lipid nanoparticle (LNP) comprising one or more of the single strands of DNA of the invention. Preferences for features of the of the single strand of DNA are as set out elsewhere herein. In some embodiments of the LNP, the single strand of DNA has a length greater than 1kb, 2kb, 3kb, 4kb, 5kb, 6kb, 7kb, 8kb, 9kb, 10kb, or more.

In some embodiments the single strand of DNA is not contained within a viral vector, for example is not contained with an adenovirus, and AAV and/or a lentivirus.

In some embodiments the single strand of DNA is contained within a viral vector, for example is contained with an adenovirus, and AAV and/or a lentivirus.

The invention also provides various nucleic acid based nanostructures that comprise one or more single strand of DNA of the invention. Nucleic acid nanostructures that are capable of expressing genes are described in WO2024105115. For example the invention provides a nucleic acid nanostructure comprising at least one scaffold strand and a plurality of staple strands, wherein said nanostructure comprises at least one single strand of DNA according to the invention.

Preferences for features of the nucleic acid based nanostructure are as defined elsewhere herein.

In some embodiments said nucleic acid nanostructure comprises a first subunit and a second subunit; wherein, said first subunit and said second subunit each comprise a single strand of DNA according to the invention, for example wherein the nucleic acid nanostructure comprises at least 2, 3, 4, 5, 6, 7, 8, 9, 10, 11, 12, 13, 14, 15, 16, 17, 18, 19, 20 or more subunits, for example where each subunit comprises a single strand of DNA of the invention.

The invention also provides a LNP comprising the nucleic acid nanostructure according to the invention.

As will be appreciated various of the single strand DNA of the invention, LNPs, dsDNA template vectors and other components described herein may be provided as a composition. Accordingly the invention provides a composition comprising one or more single strands of DNA according to the invention, the LNP to the invention, and/or the nucleic acid nanostructure according to the invention.

In some embodiments the composition is formulated for administration to a subject, for example wherein the subject is a mammal, for example a human. In some embodiments the composition is a pharmaceutical composition. In some embodiments the composition or pharmaceutical composition further comprises one or more pharmaceutically acceptable carrier and/or excipients. In the same or different embodiments the composition or pharmaceutical composition further comprises an adjuvant.

In some embodiments the composition or pharmaceutical composition further comprises components required for Nanojet administration.

In some embodiments the composition or pharmaceutical composition is formulated for delivery of the one or more single strands of DNA according to the invention, the LNP according to the invention, and/or the nucleic acid nanostructure according the invention to an immune cell, for example to a dendritic cell or a macrophage.

In some embodiments the composition or pharmaceutical composition does not comprise a viral vector, for example does not comprise an adenovirus, and AAV and/or a lentivirus.

As described herein, the single strand of DNA provided herein may suitably be used as a vaccine, therapeutic, and/or prophylactic, in the treatment of or vaccination against one or more disease(s) disclosed herein. Accordingly, the LNP provided herein, nucleic acid nanostructure provided herein, and/or composition provided herein may suitably be used as a vaccine, therapeutic, and/or prophylactic, in the treatment of or vaccination against one or more disease(s) disclosed herein.

Accordingly, provided herein is a vaccine comprising one or more single strand of DNA provided herein, the LNP provided herein, the nucleic acid nanostructure provided herein, and/or the composition provided herein.

Also provided herein is the single strand of DNA provided herein, the LNP provide herein, the nucleic acid nanostructure provided herein, the composition provided herein, and/or the vaccine provided herein for use in a method of treating or preventing an infection with a pathogen, for example a viral, bacterial, fungal or protist pathogen in a subject.

The invention also provides a method of treating or preventing and infection with a pathogen, for example a viral, bacterial, fungal or protist pathogen in a subject said method comprising administering the single strand of DNA provided herein, the LNP provided herein, the nucleic acid nanostructure provided herein the composition provided herein, and/or the vaccine provided herein to the subject.

The invention also provides the use of a single strand of DNA provided herein, the LNP provided herein, the nucleic acid nanostructure provided herein, the composition provided herein and/or the vaccine provided herein for the manufacture of a medicament for the treatment or preventing and infection with a pathogen, for example a viral, bacterial, fungal or protist pathogen in a subject.

The invention also provides the single strand of DNA provided herein, the LNP provided herein, the single strand of DNA nanostructure provided herein, the composition provided herein and/or the vaccine provided herein for use in a method of treating or preventing a disease in a subject.

The invention also provides a method of treating or preventing a disease in a subject wherein said method comprises administering a single strand of DNA provided herein, the LNP provided herein, the single strand of DNA nanostructure provided herein, the composition according provided herein and/or the vaccine provided herein to a subject.

The invention also provides the use of a single strand of DNA provided herein, the LNP provided herein, the single strand of DNA nanostructure provided herein, the composition provided herein and/or the vaccine provided herein for the manufacture of a medicament for the treatment or preventing and infection with a disease in a subject.

In some embodiments of the single strand of DNA, LNP, single strand of DNA nanostructure, composition or vaccine for use, method, or use , the first target sequence is:
a) an antisense strand that encodes a first protein or peptide and is able to be directly transcribed to produce the first RNA molecule or
b) a sense strand that is able to be transcribed to produce the first RNA molecule once the single strand of DNA is replicated to produce the antisense strand;
and wherein the first protein or peptide is a therapeutic protein or peptide.

The invention also provides the single strand of DNA provided herein, the LNP provided herein, the nucleic acid nanostructure provided herein, the LNP provided herein, the composition provided herein and/or the vaccine provided herein for use in a method of treating or preventing cancer in a subject.

The invention also provides a method of treating or preventing cancer in a subject wherein said method comprises administering a single strand of DNA provided herein, the LNP provided herein, the single strand of DNA nanostructure provided herein, the LNP provided herein, the composition provided herein and/or the vaccine provided herein to a subject.

The invention also provides the use of a single strand of DNA provided herein, the LNP provided herein, the single strand of DNA nanostructure provided herein, the composition provided herein and/or the vaccine provided herein for the manufacture of a medicament for the treatment or prevention of cancer in a subject.

In some embodiments, of the single strand of DNA, LNP, single strand of DNA nanostructure, composition or vaccine for use provided herein, method provided herein, or use provided herein, the first target sequence is:
a) an antisense strand that encodes a first protein or peptide and is able to be directly transcribed to produce the first RNA molecule or
b) a sense strand that is able to be transcribed to produce the first RNA molecule once the single strand of DNA is replicated to produce the antisense strand;

and wherein the first protein or peptide is an antigenic protein or peptide or comprises an antigenic protein or peptide,
and wherein the antigenic protein or peptide is a cancer antigen or a cancer neoantigen.

Also provided herein is a method of producing a single strand of DNA provided herein, wherein said method comprises:
a) providing a dsDNA template vector that is a phagemid and that comprises:
   i) at least one copy of the single strand of DNA provided herein wherein the one or each copy of the single strand of DNA is flanked by a self-cleaving DNA sequence, to produce a precursor template ssDNA;
   ii) component(s) ensuring propagation of the phagemid during cell division;
   iii) a selection marker, typically an antibiotic resistance gene; and
   iv) a packaging sequence,
   v) and for example one or more phage genes, that encode for phage proteins (GenIII);
b) introducing the dsDNA template vector into a microbial cell either as a double strand, or as a single stranded DNA packaged in a bacterial phage protein coat, for example an E. coli cell;
c) culturing the microbial cell under conditions so as to produce a population of cells and allow the cells to produce phage comprising the at least one copy of the single strand of DNA provided herein that are released from the cells; and
d) harvesting the phage from the culture media; and
e) extracting the ssDNA from the phage and exposing said ssDNA to conditions so as to allow self-cleavage of the self-cleaving DNA sequence;
f) isolating the single strand of DNA provided herein;
and wherein the cell also expresses:
i) genes encoding the proteins of a bacteriophage, for example genes encoding the proteins of M13 bacteriophage, for example wherein said genes are present on a helper plasmid; and
ii) for example component(s) ensuring propagation of the helper plasmid, when present, during cell division.

Also provided herein is a method of producing a single strand of DNA provided herein, wherein said method comprises:
Step (a) culturing a microbial cell that comprises:
i) at least one copy of the single strand of DNA provided herein present on a phagemid, wherein the one or each copy of the single strand of DNA is flanked by a self-cleaving DNA sequence, to produce a precursor template ssDNA, wherein the phagemid comprises a selection marker, for example an antibiotic resistance gene, and a packaging sequence; and
ii) one or more phage genes, that encode for phage proteins (GenIII); and
iii) genes encoding the proteins of a bacteriophage, for example genes encoding the proteins of M13 bacteriophage, for example wherein said genes are present on a helper plasmid

under conditions so as to produce a population of cells and allow the cells to produce phage comprising the at least one copy of the single strand of DNA provided herein that are released from the cells.

The invention also provides a method of producing the single strand of DNA of the invention wherein said method comprises:
Step (a) culturing a microbial cell that comprises:
   i) at least one copy of the single strand of DNA of the invention present on a phagemid, wherein the one or each copy of the single strand of DNA is flanked by a self-cleaving DNA sequence, to produce a precursor template ssDNA, wherein the phagemid comprises a packaging sequence;
   ii) one or more phage genes, that encode for phage proteins (GenIII); and
   iii) genes encoding the proteins of a bacteriophage, optionally genes encoding the proteins of M13 bacteriophage, optionally wherein said genes are present on a helper plasmid;
   and optionally comprises a selection marker, optionally an antibiotic resistance marker;
wherein said culturing is under conditions so as to produce a population of cells and allow the cells to produce phage comprising the at least one copy of the single strand of DNA of the invention that are released from the cells;
step (b) harvesting the phage from the culture media;
step (c) extracting the ssDNA from the phage and exposing said ssDNA to conditions so as to allow self-cleavage of the self-cleaving DNA sequence; and
step (d) isolating the single strand of DNA of the invention;
and wherein steps (a), (b), (c) and (d) are completed within a 48 hour, 72 hour, or 96 hour, 108 hour, 120 hour, 132 hour, 144 hour, 156 hour, 168 hour, 180 hour period and produce:
i) 50g, 75g, 100g, 125g, 150g, 175g, 200g of isolated single strand of DNA of the invention 1-18; and/or
ii) 50mg/L, 75mg/L, 100mg/L, 125mg/L, 150mg/L, 175mg/L, 200mg/L of isolated single strand of DNA of the invention.

The invention also provides a method of producing nucleic acid for use in a vaccine wherein the method produces:
i) 50g, 75g, 100g, 125g, 150g, 175g, 200g of isolated single strands of DNA; and/or
ii) 50mg/L, 75mg/L, 100mg/L, 125mg/L, 150mg/L, 175mg/L, 200mg/L of isolated single strands of DNA;
in a time period of 48 hours, 72 hours, or 96 hours, 108 hours, 120 hours, 132 hours, 144 hours, 156 hours, 168 hours, or 180 hours,
and wherein the method comprises any of the methods of producing the single strand of DNA of the invention as set out herein.

In some embodiments, the method further comprises step (b) harvesting the phage from the culture media. In some embodiments, the method further comprises step (c) extracting the ssDNA from the phage and exposing said ssDNA to conditions so as to allow self-cleavage of the self-cleaving DNA sequence. In some embodiments, the method further comprises step (d) isolating the single strand of DNA provided herein.

A particular advantage of the invention is that the methods provided herein may be used to generate a large quantity of a single strand of DNA or ssDNA in a short period of time, for example in a shorter period of time than existing methods. Accordingly, in some embodiments, steps (a); (a) and (b); (a), (b) and (c); or (a), (b), (c) and (d) are completed within a 48 hour, 72 hour, or 96 hour, 108 hour, 120 hour, 132 hour, 144 hour, 156 hour, 168 hour, 180 hour period.

In some embodiments:
a) 50g, 75g, 100g, 125g, 150g, 175g, 200g of isolated single strand of DNA is produced; and/or b) 50mg/L, 75mg/L, 100mg/L, 125mg/L, 150mg/L, 175mg/L, 200mg/L of isolated single strand of DNA is produced.

In some embodiments: a) 50g, 75g, 100g, 125g, 150g, 175g, 200g of isolated single strand of DNA is produced; and/or b) 50mg/L, 75mg/L, 100mg/L, 125mg/L, 150mg/L, 175mg/L, 200mg/L of isolated single strand of DNA is produced in a 48 hour, 72 hour, or 96 hour, 108 hour, 120 hour, 132 hour, 144 hour, 156 hour, 168 hour, 180 hour period.

The invention also provides a method of producing a vaccine comprising one or more single strands of DNA of the invention wherein said method comprises producing a single strand of DNA of the invention as set out herein, and formulating the isolated single strand of DNA of step (f) as a vaccine.

The invention also provides a method of producing a LNP vaccine comprising one or more single strand of DNA provided herein, wherein said method comprises producing a single strand of DNA provided herein according to a method as provided herein, and combining the isolated single strand of DNA of step (f) with lipids.

Preferences and options for a given aspect, feature or parameter of the invention should, unless the context indicates otherwise, be regarded as having been disclosed in combination with any and all preferences and options for all other aspects, features and parameters of the invention. For example, the invention provides:
a) an antisense single strand of DNA that encodes three different antigens from three different strains of influenza;
b) a linear single strand of DNA that encodes a antigen from a first bacterial species wherein said antigen is fused to ubiquitin and also encodes an antigen from a second bacterial species wherein said antigen from the second bacterial species is fused to a chemokine ligand;
c) a LNP comprising a circular single strand of DNA of the invention, wherein the single strand of DNA of the invention comprises a first sequence that encodes a Cas protein at three or more sequences that encode different gRNAs, allowing for targeting of the Cas protein to at least three different loci in a host;
d) a composition formulated for nanojet administration wherein the composition comprises a single strand of DNA of the invention, and wherein the first sequence encodes a viral antigen.

In this way the skilled person will appreciate how different features from different passages within this text can be combined together.

### The invention is also illustrated in the following numbered paragraphs:

1. A single strand of DNA that:
   comprises a first target sequence operably linked to a first promoter sequence, wherein the first promoter sequence drives expression of the first target sequence to produce a first RNA molecule either:
   i) directly from the single strand of DNA; or
   ii) following replication of the single strand of DNA into the double strand form.
2. The single strand of DNA of paragraph 1 wherein the single strand of DNA is entirely single stranded and/or is substantially single stranded.
3. The single strand of DNA of any of paragraphs 1 or 2 wherein the single strand of DNA contains no regions of self-complementarity, optionally no regions of self-complementarity of sufficient length so as to allow internal hybridisation, optionally no regions of self-complementarity:
   a) that are longer than 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or 100 base pairs; and/or
   b) where the combined length of two regions of the single strand of DNA that make up a particular self-complementary region constitute less than 50%, 45%, 40%, 35%, 30%, 25%, 20%, 15%, 10%, 5%, or 2% or less than 1% of the total length of the single strand of DNA.
4. The single strand of DNA of paragraph 1 wherein the single strand of DNA comprises at least one region that is double stranded, wherein said double stranded region is generated via hybridisation between at least a first and second region of the single strand of DNA that are substantially complementary to each other, optionally:
   a) wherein the hybridisation between a first and second region of the single stand of DNA enhances expression of the first RNA molecule, optionally wherein the first region of the single strand of DNA is in the promoter and the second region of the single strand of DNA is distal to the promoter; or
   b) wherein the first and second region of the single strand of DNA that are substantially complementary to each other are located at the 5' and the 3' end of the single strand of DNA so that the single strand of DNA is circularised upon hybridisation between the first and second regions.
5. The single strand of DNA of any one of paragraphs 1-4 wherein the single strand of DNA comprises a hairpin, optionally a hairpin at the 5' and/or 3' end of the single strand of DNA.
6. The single strand of DNA of paragraph 1, 4 or 5 wherein the single strand of DNA comprises a secondary structure so as to:
   a) increase stability of the single stand of DNA; and/or
   b) to increase expression from the promoter.
7. The single strand of DNA of any of the preceding paragraphs wherein the promoter is a promoter capable of driving transcription in a eukaryotic cell, optionally in a mammalian cell, optionally in a human cell, optionally in a dendritic cell, T cell and/or B cell.
8. The single strand of DNA of any of the preceding paragraphs wherein the promoter is a constitutive promoter.
9. The single strand of DNA of any of the preceding paragraphs wherein the promoter is an inducible promoter.
10. The single strand of DNA of any of the preceding paragraphs wherein the promoter is selected from the group comprising or consisting of: Cytomegalovirus (CMV) Immediate-Early promoter; Simian Virus 40 (SV40) promoter; Muscle Creatine Kinase promoter (MCK); or a tissue or cell-type specific promoter, optionally dendritic cell, B cell and/or T cell specific promoter.
11. The single strand of DNA of any of the preceding paragraphs wherein the promoter is selected from the group comprising or consisting of: Human Elongation Factor 1 Alpha (EF1a) Promoter; Tetracycline-Inducible Promoter; a MiniPromoter; or a tissue or cell-type specific promoter.
12. The single strand of DNA of any of the preceding paragraphs wherein the promoter is selected from the group comprising or consisting of: U6 promoter; H1 promoter; or a CMV promoter; or a tissue or cell-type specific promoter.
13. The single strand of DNA of any of paragraphs 1-12 further comprising a first enhancer sequence, wherein the first enhancer sequence enhances expression of the first target sequence, optionally enhances expression of the first target sequence in a specific cell type, optionally wherein at least one enhancer sequence is located in the 5'UTR, 3'UTR or in the 5'UTR and 3'UTR;
   optionally wherein the enhancer is selected from the group comprising or consisting of:
   a) a viral enhancer, optionally the 72-bp enhancer element from Simian Virus 40 (SV40), the enhancer from CMV, and/or the enhancer from Rous sarcoma virus;
   b) a Ubiquitous/Constitutive Enhancers, optionally the enhancer from the human elongation factor 1 alpha (EF1a) enhancer;
   c) a tissue/Cell-Type Specific Enhancers, optionally the muscle creatine kinase (MCK) enhancer for muscle cells;
   d) Super-enhancers associated with genes defining cell identity;
   e) Synthetic/Designed Enhancers
   f) inducible enhancers, optionally an inducible enhancer that response to hormones or small molecules;
   optionally wherein the single strand of DNA comprises more than one enhancer, optionally comprises combinations of at least two enhancers, optionally at least two enhancers of any of (a)-(f), optionally a combination of the SV40 enhancer with a muscle-specific enhancer for muscle targeting.
14. The single strand of DNA according to any of the preceding paragraphs further comprising any one or more of:
   a) a nuclear localisation signal; and/or
   b) inverted-terminal repeat like hairpin motifs.
15. The single strand of DNA of any of the preceding paragraphs wherein the single strand of DNA is linear, optionally wherein the single strand of DNA comprises caps formed by hairpin loops at the 5' end and/or the 3' end.
16. The single strand of DNA of any of paragraphs 1-14 wherein the single strand of DNA is circular, optionally wherein in the single strand of DNA is circularised via complementary base pairing of the 5' end and the 3' end.
17. The single strand of DNA of any of paragraphs 1-15 wherein the single strand of DNA is linear, and capable of circularising when in a cellular environment, optionally a eukaryotic cell, optionally a mammalian cell, optionally a human cell; and or is capable of circularising in an in vitro transcription translation system, optionally in a mammalian in vitro transcription translation system.
18. The single strand of DNA of any of the preceding paragraphs wherein the single strand of DNA comprises any one or more modifications, optionally:
   a) a modification to reduce immunogenicity, optionally reduce immunogenicity in a mammalian cell, optionally wherein the modification is methylation;
   b) a modification to enhance expression, optionally enhance expression in a mammalian cell, optionally wherein the modification comprises or consists of any one or more of:
   c) a modification to make the single strand of DNA act as an adjuvant, optionally wherein the modification is non-methylation.
19. The single strand of DNA of any of the preceding paragraphs wherein the single strand of DNA additionally comprises a second target sequence, or a third, fourth, fifth, sixth, seventh, eighth, ninth or tenth or more target sequence.
20. The single strand of DNA of paragraph 19 wherein each target sequence is the same as the first target sequence.
21. The single strand of DNA of paragraph 19 wherein the single strand of DNA comprises at least two target sequences that are different to each other.
22. The single strand of DNA of any of paragraphs 19-21 wherein the target sequences present in the single strand of DNA are arranged in a tandem array.
23. The single strand of DNA of paragraph 22 wherein all of the target sequences present in the tandem array are operably linked to the same promoter.
24. The single strand of DNA of any of paragraphs 19-23 wherein at least two of the target sequences are operably linked to different promoters.
25. The single strand of DNA of any of paragraphs 19-24 wherein the target sequences are transcribed into a single RNA molecule.
26. The single strand of DNA of paragraph 25 wherein the single RNA molecule comprises an internal ribosome entry site (IRES) that is present 5' to each target sequence, so that each target sequence is translated, optionally wherein the IRES is an IRES from encephalomyocarditis virus (EMCV) or poliovirus.
27. The single strand of DNA of any of paragraphs 25 or 26 wherein each target sequence of the single RNA molecule comprises a start and stop codon.
28. The single strand of DNA of any of paragraphs 25 or 26 wherein spacer sequences are present between the IRES and one or more target sequences.
29. The single strand of DNA of any of the preceding paragraphs further comprising a transcription terminator.
30. The single strand of DNA according to any of paragraphs 26-29 wherein the promoter is a strong promoter, optionally a CMV promoter, SP6 promoter or T7 promoter.
31. The single strand of DNA according to any of the preceding paragraphs, wherein the single strand of DNA is:
   At least 500 nucleotides, or at least 1000 nucleotides or at least 1250, 1500, 1750, 2000, 2250, 2500, 2750, 3000, 3250, 3500, 3750, 4000, 4250, 4500, 4750, 5000, 5250, 5500, 5750, 6000, 6250, 6500, 6750, 7000, 7250, 7500, 7750, 8000, 8250, 8500, 8750, 9000, 9250, 9500, 9750, 10000, 10250, 10500, 10750, 11000, 11250, 11500, 11750, 12000, 12250, 12500, 12750, 13000, 13250, 13500, 13750, 14000, 14250, 14500, 14750, 15000, 15250, 15500, 15750, 16000, 16250, 16500, 16750, 17000, 17250, 17500, 17750, 18000, 18250, 18500, 18750, 19000, 19250, 19500, 19750, or at least 20000 and/or
   Between 1000 and 20000, 1250 and 19750, 1500 and 19500, 1750 and 19250, 2000 and 19000, 2250 and 18750, 2500 and 18500, 2750 and 18250, 3000 and 18000, 3250 and 17750, 3500 and 17500, 3750 and 17250, 4000 and 17000, 4250 and 16750, 4500 and 16500, 4750 and 16250, 5000 and 16000, 5250 and 15750, 5500 and 15500, 5750 and 15250, 6000 and 15000, 6250 and 14750, 6500 and 14500, 6750 and 14250, 7000 and 14000, 7250 and 13750, 7500 and 13500, 7750 and 13250, 8000 and 13000, 8250 and 12750, 8500 and 12500, 8750 and 12250, 9000 and 12000, 9250 and 11750, 9500 and 11500, 9750 and 11250, 10000 and 11000, 10250 and 10750 nucleotides in length.
32. The single strand of DNA of any of the preceding paragraphs wherein the single strand of DNA is designed so as to allow expression of the first target sequence to produce a first RNA molecule in eukaryotes, optionally in mammals, optionally in humans.
33. The single strand of DNA according to any of paragraphs 1-32 wherein the first target sequence is:
   a) an antisense strand that encodes a first protein or peptide and is able to be directly transcribed to produce the first RNA molecule; or
   b) a sense strand that is able to be transcribed to produce the first RNA molecule once the single strand of DNA is replicated to produce the antisense strand.
34. The single strand of DNA according to any of the preceding paragraphs wherein the first target sequence comprises one or more sequences that when transcribed into the first RNA molecule form a 5'UTR, a 3'UTR and/or a polyA tail.
35. The single strand of DNA according to paragraph 34 wherein the 5'UTR and/or 3'UTR comprise sequences that form RNA secondary structures that regulate gene expression in eukaryotes, optionally wherein:
   a) the 5'UTR comprises:
      i) one or more stable hairpin and/or stem-loop structures to downregulate gene expression; and/or
      ii) an internal ribosome entry site (IRES), optionally wherein the IRES is an IRES from encephalomyocarditis virus (EMCV) or poliovirus;
      and/or
   b) the 3'UTR comprises:
      i) one or more stem-loop structures and/or AU rich elements; and/or
      ii) one or more miRNA binding sites.
36. The single strand of DNA according to any one of paragraphs 33-35 wherein the first protein or peptide is an antigenic protein or peptide or comprises an antigenic protein or peptide.
37. The single strand of DNA according to paragraph 36 wherein the first protein or peptide that is an antigenic protein or peptide or comprises an antigen protein or peptide comprises one or more sequences to enhance presentation of the antigen protein or peptide or fragments thereof, optionally:
   a) presentation by the major histocompatibility proteins (MHC), optionally MHC I and/or MHC II; and/or
   b) presentation by antigen presenting cells, optionally dendritic cells, macrophages and/or B cells.
38. The single strand of DNA according to paragraph 37 wherein the one or more sequences to enhance presentation of the first protein or peptide fragment is:
   a) a ubiquitin sequence;
   b) an Endoplasmic Reticulum (ER) signal peptide, optionally the signal peptide from tissue plasminogen activator (tPA);
   c) the Lysosome-Associated Membrane Protein 1 (LAMP-1); and/or
   d) a chemokine ligand, optionally XCL1.
39. The single strand of DNA according to any of paragraphs 33-38 wherein the first protein or peptide is a protein or peptide present in a pathogen, optionally wherein the pathogen is a bacterial pathogen, viral pathogen, fungal pathogen or a protist pathogen.
40. The single strand of DNA according to any of paragraphs 33-39 wherein the first protein or peptide is a cancer antigen or a cancer neoantigen.
41. The single strand of DNA according to any of paragraphs 33-40 wherein the first protein or peptide is:
   a) a viral antigen, optionally
      i) an influenza antigen, optionally Influenza Hemagglutinin, optionally from H5Nx strains;
      ii) a coronavirus antigen, optionally from SARS-CoV-2, SARS-CoV, HCoV NL63, HCoV HKU1, or MERS-CoV; optionally a spike protein, optionally the spike protein from any one or more of from SARS-CoV-2, SARS-CoV, HCoV NL63, HCoV HKU1, or MERS-CoV;
      iii) a HIV antigen, optionally HIV-1 ENV or HIV-1 GAG; or
      iv) a Hepatitis B antigen, optionally Hepatitis B Surface Antigen (HBsAg);
   b) a cancer antigen, optionally wherein the cancer antigen is NY-ESO-1, MAGE-A3, HER2/neu; or PSA;
   c) an Autoimmune Disease Antigen, optionally insulin, Myelin Basic Protein (MBP);
   d) a Neurological Disease Antigens, optionally Amyloid Beta (Aβ) or Tau Protein;
   e) a bacterial antigen, optionally
      i) an antigen from *Mycobacterium tuberculosis,* optionally Ag85 Complex, optionally from any ofAg85A, Ag85B, Ag85C); ESAT-6; CFP-10
      ii) an antigen from *Bacillus anthracis,* optionally Protective Antigen (PA);
      iii) an antigen from *Clostridium tetani,* optionally tetanus toxin (TeNT)
      iv) an antigen from *Escherichia coli,* optionally Colonization Factor Antigen I (CFA/I);
      v) *Salmonella enterica,* optionally Flagellin (FliC), Invasin Proteins (e.g., SipA, SipC);
      vi) *Streptococcus pneumoniae,* optionally Pneumolysin (Ply), PspA (Pneumococcal Surface Protein A);
      vii) *Helicobacter pylori,* optionally Urease Subunits (UreA, UreB);
      viii) *Listeria monocytogenes,* optionally Listeriolysin O (LLO);
      ix) *Shigella* spp., optionally Ipa Proteins (e.g., IpaB, IpaC);
      x) *Vibrio cholerae,* optionally Cholera Toxin (CT), optionally the B subunit of cholera toxin (CTB);
   f) a fungal antigen, optionally
      i) *Candida albicans,* optionally Agglutinin-like sequence 3 (Als3), Secreted aspartyl proteinases (Sap), Heat shock protein 90 (Hsp90), Hyphal wall protein 1 (Hwp1), or Enolase (Enol);
      ii) *Aspergillus fumigatus,* optionally Aspf3 (Allergen), Catalase, Gel1 (1,3-β-glucanosyltransferase), or Pmp20 (Putative Plasma Membrane Protein);
      iii) *Cryptococcus neoformans,* optionally Glucosylceramide (GlcCer), Mannoproteins (MP98, MP88), Phospholipase B1 (Plb1), or Capsular polysaccharide (GXM);
      iv) *Coccidioides posadasii*/*immitis,* optionally Antigen 2/PRA (Proline-rich antigen), Chitinase, Urease, Fba (Fructose-bisphosphate aldolase),
      v) *Histoplasma capsulatum,* optionally Histone 2B (H2B), Heat shock protein 60 (Hsp60), Catalase B (CatB), Yps3 (Yeast protein 3);
      vi) *Paracoccidioides brasiliensis,* optionally Glycoprotein 43 (Gp43) peptide 10 (P10), Paracoccin, Triosephosphate isomerase (Tpi),
      vii) *Pneumocystis jirovecii,* optionally Major surface glycoprotein (Msg), Kexin, Monosaccharide transporter (Mst);
      and/or
   g) a protist antigen.
42. The single strand of DNA according to any of paragraphs 1-41 wherein the single strand of DNA comprises at least one adjuvant sequence that encodes an adjuvant protein, optionally wherein the adjuvant protein is selected from the group comprising or consisting of:
   a) Bacterial protein adjuvants, optionally FomA porin from Fusobacterium; MOMP from *Shigella flexneri,* Porin from *Shigella dysenteriae;* OmpU from *Vibrio cholerae*; PorB from Neisseria meningitidis; OmpC and OmpF from *Salmonella typhi,* ESAT-6 from *Mycobaceterium tuberculosis*; rBCSP31 from *Brucella abortus*; DnaJ-ΔA146Ply from *Streptococcus pneumoniae*; Endopeptidase O from *Streptococcus pneumoniae*; Cholera toxin from *Vibrio cholerae*; B-pentamers of LT-IIa and LT-IIb from *Escherichia coli*; Omp16 from *Brucella abortus*; BLS from *Brucella* spp.; GrpE from *M. tuberculosis*; RpfE from M. *tuberculosis*; Rv0652 from *M. tuberculosis*; HBHA from *M. tuberculosis*; P97 protein from *Mycoplasma hyopneumoniae*; Flagellin from *Salmonella* species; Entolimod (CBLB502) optimised from *Salmonella* flagellin;
   b) One or more cytokines, optionally wherein the cytokine is selected from the group comprising or consisting of Interleukin-12 (IL-12), Interleukin-2 (IL-2), Granulocyte-Macrophage Colony-Stimulating Factor (GM-CSF);
   c) one or more cytokines, optionally wherein the chemokine is CCL21 (Secondary Lymphoid Tissue Chemokine, SLC);
   d) one or more co-stimulatory molecules, optionally wherein the co-stimulatory molecule is CD40 Ligand (CD40L), or B7-1 (CD80) and B7-2 (CD86);
   e) one or more Toll-Like Receptor (TLR) Ligands, optionally wherein the Toll-Like Receptor (TLR) Ligand is CpG Oligodeoxynucleotides (CpG ODNs)
   f) Flt3 Ligand (Flt3L); and/or
   g) Heat Shock Proteins (HSPs)
   or any combinations thereof.
43. The single strand of DNA according to any one of paragraphs 33-42 wherein the first protein or peptide is a therapeutic protein or peptide.
44. The single strand of DNA of paragraph 43 wherein the therapeutic protein or peptide is capable of replacing a defective or deficient protein associated with a genetic disorder.
45. The single strand of DNA of paragraph 44 wherein the genetic disorder is selected from the group comprising or consisting of:
   adenosine deaminase severe combined immunodeficiency (ADA-SCID), neuronal ceroid lipofuscinosis, aspartylglucosaminuria, Batten disease, beta-thalassemia, chronic granulomatous disease (CGD), cystic fibrosis, cystinosis, Duchenne muscular dystrophy, Fabry disease, Gaucher disease type I, Gaucher disease type II, Gaucher disease type III, Huntingdon's disease, Hurler disease, Leber's congenital amaurosis, Maroteau-Lamy disease, metachromatic leukodystrophy, Morquio disease type A, Morquio disease type B, mucolipidosis type I, mucolipidosis type II, mucolipidosis type III, mucolipidosis type IV, Niemann-Pick disease type A, Niemann-Pick disease type B, Niemann-Pick disease type C1, Niemann-Pick disease type C2, Pompe disease, retinitis pigmentosa, Sandhoff disease, sickle cell disease, Spinal Muscular Atrophy, X-ALD, hemophilia A, hemophilia B, Hunter disease (MPS II), lysosomal storage disorder, Maroteaux-Lamy disease (MPS VI), Neuronal ceroid lipofuscinosis (NCL) including CLN1 disease, Sanfilippo disease type A (MPS IIIA), Sanfilippo disease type B (MPS IIIB), Sanfilippo disease type C (MPS IIIC), Sanfilippo disease type D (MPS IIID), Schindler disease type I, Schindler disease type II, Sly disease (MPS VII), Tay-Sachs disease.
46. The single strand of DNA according to any of paragraphs 1-32 wherein the first target sequence is a non-coding RNA, optionally a functional RNA, optionally selected from the group comprising or consisting of:
   a) a gRNA;
   b) an RNA designed to have sequence complementarity to a host target RNA so as to cause RNAi when expressed in a host cell, optionally a miRNA; and/or
   c) a IncRNA.
47. The single strand of DNA of paragraph 46 wherein the RNA is designed to have sequence complementarity to a host target RNA that is RNA transcribed from an oncogene, optionally wherein the oncogene is selected from the group comprising or consisting of KRAS, EGFR (Epidermal Growth Factor Receptor), CFTR (Cystic Fibrosis Transmembrane Conductance Regulator), DMD (Dystriophin).
48. The single strand of DNA according to any of paragraphs 1-47 wherein the single strand of DNA comprises at least a first target sequence that encodes a protein or peptide, and at least a second target sequence that is not a protein or peptide coding sequence and wherein the RNA is a functional RNA.
49. The single strand of DNA according to paragraph 48 wherein the protein or peptide is a Cas protein, and wherein the RNA is a corresponding gRNA.
50. The single strand of DNA of paragraph 49, wherein the gRNA is designed to have sequence complementarity to a viral genome or viral nucleic acid, optionally wherein the virus is selected from the group comprising or consisting of HBV optionally wherein the viral nucleic acid is cccDNA (Covalently Closed Circular DNA), or HIV Provirus.
51. The single strand of DNA according to any of paragraphs 19-50 wherein the single strand of DNA comprises a second target sequence, or a third, fourth, fifth, sixth, sevenths, eighth, ninth or tenth or more target sequence, and wherein each target sequence encodes, or encodes following replication of the single strand of DNA, any one or more of:
   a) a protein or peptide that is an antigenic protein or peptide or comprises an antigenic protein or peptide;
   b) a protein or peptide that is a therapeutic protein or peptide;
   c) an adjuvant protein or peptide; and/or
   d) a non-coding RNA, optionally a functional RNA, optionally selected from the group comprising or consisting of:
      i) a gRNA;
      ii) an RNA designed to have sequence complementarity to a host target RNA so as to cause RNAi when expressed in a host cell; and/or
      iii) a IncRNA.
52. The single strand of DNA according to paragraph 51 wherein the single strand of DNA comprises 2, 3, 4, 5, 6, 7, 8, 9, 10 or more target sequences that each encodes, or encodes following replication of the single strand of DNA, an antigenic protein or peptide.
53. The single strand of DNA according to paragraph 51 or 52 wherein at least two of the target sequences encode, or encodes following replication of the single strand of DNA, different antigenic proteins or peptides, optionally at least 3, 4, 5, 6, 7, 8, 9 or at least 10 of the target sequences encode, or encode following replication of the single strand of DNA, different antigenic proteins or peptides.
54. The single strand of DNA according to paragraph 51-53 wherein at least two of the target sequence encode, or encode following replication of the single strand of DNA, the same antigenic protein or peptide, optionally wherein at least 3, 4, 5, 6, 7, 8, 9 or at least 10 of the target sequences encode, or encode following replication of the single strand of DNA, the same antigenic proteins or peptides.
55. The single strand of DNA according to any of paragraphs 51-54 wherein at least two of the target sequences encode, or encode following replication of the single strand of DNA, an antigenic protein or peptide that each comprise one or more sequences to enhance presentation of the protein or peptide or fragments thereof,
   and wherein at least two of the antigenic proteins or peptides comprise different sequences to enhance presentation of the protein or peptide or fragments thereof, optionally wherein the sequences to enhance presentation of the protein or peptide or fragments thereof are selected from the group comprising or consisting of:
   a) a ubiquitin sequence;
   b) an Endoplasmic Reticulum (ER) signal peptide, optionally the signal peptide from tissue plasminogen activator (tPA);
   c) the Lysosome-Associated Membrane Protein 1 (LAMP-1); and/or
   d) a chemokine ligand, optionally XCL1.
56. The single strand of DNA according to any of paragraphs 51-55 wherein at least two of the target sequences encode, or encode following replication of the single strand of DNA, the same antigenic protein or peptide and each protein or peptide comprises one or more sequences to enhance presentation of the first protein or peptide or fragments thereof, but wherein the one or more sequences to enhance presentation of the first protein or peptide or fragments thereof present in the first target sequence are different to those of the second target sequence.
57. The single strand of DNA of any of the preceding paragraphs wherein the single strand of DNA is not a closed linear DNA.
58. The single strand of DNA of any of the preceding paragraphs wherein the single strand of DNA is not doggybone DNA (dbDNA).
59. The single strand of DNA of any of the preceding paragraphs wherein the single strand of DNA does not comprise a telomerase target sequence or a protelomerase target sequence.
60. The single strand of DNA of any of the preceding paragraphs wherein the single strand of DNA does not comprise a hairpin loop at the 5', 3', or 5' and 3' termini.
61. The single strand of DNA of any of paragraphs 1-60 wherein the single strand of DNA was produced by expressing ssDNA from a dsDNA template vector that comprises at least one copy of the single strand of DNA of any of paragraphs 1-60, wherein the one or each copy of the single strand of DNA is flanked by a self-cleaving DNA sequence, to produce a precursor template ssDNA, wherein said expression is in a microbial cell culture, optionally:
   a) a bacterial cell culture, optionally an *E. coli* cell, optionally a K12-derived *E. coli* safety strain, optionally DH5alpha (DH5a), XL-1blue or JM109; or
   b) a yeast cell culture.
62. The single strand of DNA of paragraph 61 wherein the dsDNA template vector is a phagemid, optionally further comprising:
   a) a packaging sequence;
   b) component(s) ensuring propagation of the phagemid during cell division; and/or
   c) a selection marker, typically an antibiotic resistance gene.
63. The single strand of DNA of paragraph 61 or 62 wherein following expression of the precursor template ssDNA from the dsDNA template vector, the ssDNA is isolated from the microbial cell or cell culture media.
64. The single strand of DNA of any of paragraphs 61-63 wherein the precursor template ssDNA is digested under reaction conditions where the self-cleaving DNA sequences become active to produce the single strand of DNA , optionally wherein said conditions comprise the addition of Zn²⁺.
65. The single strand of DNA of any of paragraphs 61-64 wherein the self-cleaving DNA sequences are self-cleaving deoxyribozymes or DNAzymes, optionally Zn²⁺-dependent DNAzymes, optionally I-R3.
66. The single strand of DNA of any of paragraphs 61-65 wherein the dsDNA template vector comprises two, three, four or more, single strand of DNA s as defined in any of paragraphs 1-60.
67. The single strand of DNA of any of paragraphs 61-66 wherein expression of ssDNA from the dsDNA template vector occurs in the presence of a helper plasmid or a helper phage which comprises:
   a) genes encoding the proteins of a bacteriophage, *e.g.,* M13 bacteriophage;
   b) component(s) ensuring propagation of the helper plasmid during cell division; and/or
   c) a selection marker, optionally an antibiotic resistance gene.
68. The single strand of DNA of any of paragraphs 61-67 wherein the microbial cell is a bacterial cell and the single strand of DNA is packaged into phage-like particles which are secreted from the bacterial cells, and wherein the phage-like particles are isolated from the cell culture and the phagemid ssDNA is purified from the phage-like particles.
69. The single strand of DNA of any of paragraphs 1-68 wherein the single strand of DNA further comprises fixed 5' and 3' end sequences, optionally wherein the fixed 5' and 3' end sequences are the scars that remain following DNAzyme cleavage.
70. The single strand of DNA of any of the preceding paragraphs wherein the single strand of DNA is stable at a temperature of at least 18°C, or at least 19°C, 20°C, 21°C, 22°C, 23°C, 24°C, 25°C, or 26°C, optionally stable for at least 24 hours, 48 hours, 1 week, 2 weeks, 4 weeks, 2 months, 6 months, 1 year or more.
71. A dsDNA vector comprising a sequence corresponding to the single strand of DNA of any of paragraphs 1-70.
72. The dsDNA vector of paragraph 71 wherein the vector comprises multiple repeats of the sequence corresponding to the single strand of DNA of any of paragraphs 1-60, optionally wherein the multiple repeats are arranged in a head-to-tail orientation, optionally wherein the dsDNA vector comprises at least 2, 3, 4, 5, 6, 7, 8, 9, 10, 11, 12, 13, 14, 15, 16, 17, 18, 19, 20, 25, 30, 35, 40, 45, 50 or more repeats.
73. The dsDNA vector of paragraph 52 wherein the vector is a phagemid, and optionally further comprises:
   a) a packaging sequence;
   b) component(s) ensuring propagation of the phagemid during cell division; and/or
   c) a selection marker, typically an antibiotic resistance gene.
74. The dsDNA vector of paragraph 73 wherein the phagemid further comprises:
   a) genes encoding the proteins or parts of a protein of a bacteriophage, optionally proteins or parts of a protein of the M13 bacteriophage;
   b) component(s) ensuring propagation of the helper plasmid during cell division; and/or
   c) a selection marker, optionally an antibiotic resistance gene.
75. A lipid nanoparticle (LNP) comprising one or more of the single strand of DNA of any one of paragraphs 1-70.
76. The LNP of paragraph 75 wherein the single strand of DNA has a length greater than 1kb, 2kb, 3kb, 4kb, 5kb, 6kb, 7kb, 8kb, 9kb, 10kb, or more.
77. The single strand of DNA according to any of paragraphs 1-70 wherein the single strand of DNA is not contained within a viral vector, optionally is not contained with an adenovirus, and AAV and/or a lentivirus.
78. A nucleic acid nanostructure comprising at least one scaffold strand and a plurality of staple strands, wherein said nanostructure comprises at least one single strand of DNA according to any of paragraphs 1-70.
79. The nucleic acid nanostructure according to paragraph 78 wherein said nucleic acid nanostructure comprises a first subunit and a second subunit; wherein, said first subunit and said second subunit each comprise a single strand of DNA according to any of paragraphs 1-51, optionally wherein the nucleic acid nanostructure comprises at least 2, 3, 4, 5, 6, 7, 8, 9, 10, 11, 12, 13, 14, 15, 16, 17, 18, 19, 20 or more subunits, optionally where each subunit comprises a single strand of DNA according to any of paragraphs 1-70.
80. A LNP comprising the nucleic acid nanostructure according to any of paragraphs 78-79.
81. A composition comprising one or more single strand of DNA s according to any of paragraphs 1-70, the LNP of any of paragraphs 75 or 76, and/or the nucleic acid nanostructure according to any of paragraphs 78-79.
82. The composition according to paragraph 81 wherein the composition is formulated for administration to a subject, optionally wherein the subject is a mammal, optionally a human.
83. The composition according to any of paragraphs 81 or 82 further comprising a pharmaceutically acceptable carrier and/or excipients.
84. The composition according to any of paragraphs 81-83 further comprising an adjuvant.
85. The composition according to any of paragraphs 81-84 further comprising components required for Nanojet administration.
86. The composition according to any of paragraphs 81-85 wherein the composition is formulated for delivery of the one or more single strand of DNA s according to any of paragraphs 1-70, the LNP of any of paragraphs 75 or 76, and/or the nucleic acid nanostructure according to any of paragraphs 78-79 to an immune cell, optionally to a dendritic cell or a macrophage.
87. The composition according to any of paragraphs 81-86 wherein the composition does not comprise a viral vector, optionally does not comprise an adenovirus, and AAV and/or a lentivirus.
88. A vaccine comprising one or more single strand of DNA according to any of paragraphs 1-70, the LNP of any of paragraphs 75 or 76, the nucleic acid nanostructure according to any of paragraphs 78-79, a LNP according to paragraph 80, and/or the composition according to any of paragraphs 81-87.
89. The single strand of DNA according to any of paragraphs 1-70, the LNP of any of paragraphs 75 or 76, the nucleic acid nanostructure according to any of paragraphs 78-79, a LNP according to paragraph 80, the composition according to any of paragraphs 81-87 and/or the vaccine according to paragraph 88 for use in a method of treating or preventing an infection with a pathogen, optionally a viral, bacterial, fungal or protist pathogen in a subject.
90. A method of treating or preventing and infection with a pathogen, optionally a viral, bacterial, fungal or protist pathogen in a subject said method comprising administering the single strand of DNA according to any of paragraphs 1-70, the LNP of any of paragraphs 75 or 76, the nucleic acid nanostructure according to any of paragraphs 78-79, a LNP according to paragraph 80, the composition according to any of paragraphs 81-87 and/or the vaccine according to paragraph 88 to the subject.
91. Use of a single strand of DNA according to any of paragraphs 1-70, the LNP of any of paragraphs 75 or 76, the nucleic acid nanostructure according to any of paragraphs 78-79, a LNP according to paragraph 80, the composition according to any of paragraphs 81-87 and/or the vaccine according to paragraph 88 for the manufacture of a medicament for the treatment or preventing and infection with a pathogen, optionally a viral, bacterial, fungal or protist pathogen in a subject.
92. The single strand of DNA according to any of paragraphs 1-70, the LNP of any of paragraphs 75 or 76, the single strand of DNA nanostructure according to any of paragraphs 78-79, a LNP according to paragraph 80, the composition according to any of paragraphs 81-87 and/or the vaccine according to paragraph 88 for use in a method of treating or preventing a disease in a subject.
93. A method of treating or preventing a disease in a subject wherein said method comprises administering a single strand of DNA according to any of paragraphs 1-70, the LNP of any of paragraphs 75 or 76, the single strand of DNA nanostructure according to any of paragraphs 78-79, a LNP according to paragraph 80, the composition according to any of paragraphs 81-87 and/or the vaccine according to paragraph 88 to a subject.
94. Use of a single strand of DNA according to any of paragraphs 1-70, the LNP of any of paragraphs 75 or 76, the single strand of DNA nanostructure according to any of paragraphs 78-79, a LNP according to paragraph 80, the composition according to any of paragraphs 81-87 and/or the vaccine according to paragraph 88 for the manufacture of a medicament for the treatment or preventing and infection with a disease in a subject.
95. The single strand of DNA , LNP, single strand of DNA nanostructure, composition or vaccine according to paragraph 92, method according to paragraph 93 or use according to paragraphs 94 wherein the first target sequence is:
   a) an antisense strand that encodes a first protein or peptide and is able to be directly transcribed to produce the first RNA molecule or
   b) a sense strand that is able to be transcribed to produce the first RNA molecule once the single strand of DNA is replicated to produce the antisense strand;
   and wherein the first protein or peptide is a therapeutic protein or peptide.
96. The single strand of DNA according to any of paragraphs 1-70, the LNP of any of paragraphs 75 or 76, the nucleic acid nanostructure according to any of paragraphs 78-79, a LNP according to paragraph 80, the composition according to any of paragraphs 81-87 and/or the vaccine according to paragraph 88 for use in a method of treating or preventing cancer in a subject.
97. A method of treating or preventing cancer in a subject wherein said method comprises administering a single strand of DNA according to any of paragraphs 1-70, the LNP of any of paragraphs 75 or 76, the single strand of DNA nanostructure according to any of paragraphs 78-79, a LNP according to paragraph 80, the composition according to any of paragraphs 81-87 and/or the vaccine according to paragraph 88 to a subject.
98. Use of a single strand of DNA according to any of paragraphs 1-70, the LNP of any of paragraphs 75 or 76, the single strand of DNA nanostructure according to any of paragraphs 78-79, a LNP according to paragraph 80, the composition according to any of paragraphs 81-87 and/or the vaccine according to paragraph 88 for the manufacture of a medicament for the treatment or prevention of cancer in a subject.
99. The single strand of DNA, LNP, single strand of DNA nanostructure, composition or vaccine according to paragraph 96, method according to paragraphs 97 or use according to paragraphs 98 wherein the first target sequence is:
   a) an antisense strand that encodes a first protein or peptide and is able to be directly transcribed to produce the first RNA molecule; or
   b) a sense strand that is able to be transcribed to produce the first RNA molecule once the single strand of DNA is replicated to produce the antisense strand;
      and wherein the first protein or peptide is an antigenic protein or peptide or comprises an antigenic protein or peptide,
      and wherein the antigenic protein or peptide is a cancer antigen or a cancer neoantigen.
100. A method of producing a single strand of DNA of any of paragraphs 1-70, wherein said method comprises:
   a) providing a dsDNA template vector that is a phagemid and that comprises:
      i) at least one copy of the single strand of DNA of any of paragraphs 1-70 wherein the one or each copy of the single strand of DNA is flanked by a self-cleaving DNA sequence, to produce a precursor template ssDNA;
      ii) component(s) ensuring propagation of the phagemid during cell division;
      iii) a selection marker, typically an antibiotic resistance gene; and
      iv) a packaging sequence;
         and optionally
      v) one or more phage genes, that encode for phage proteins (GenIII);
   b) introducing the dsDNA template vector into a microbial cell either as a double strand, or as a single stranded DNA packaged in a bacterial phage protein coat, optionally an *E. coli* cell;
   c) culturing the microbial cell under conditions so as to produce a population of cells and allow the cells to produce phage comprising the at least one copy of the single strand of DNA of any of paragraphs 1-70 that are released from the cells; and
   d) harvesting the phage from the culture media; and
   e) extracting the ssDNA from the phage and exposing said ssDNA to conditions so as to allow self-cleavage of the self-cleaving DNA sequence;
   f) isolating the single strand of DNA of any of paragraphs 1-70;
   and wherein the cell also expresses:
   i) genes encoding the proteins of a bacteriophage, optionally genes encoding the proteins of M13 bacteriophage, optionally wherein said genes are present on a helper plasmid; and
   ii) optionally component(s) ensuring propagation of the helper plasmid, when present, during cell division.
101. A method of producing a single strand of DNA of any of paragraphs 1-70, wherein said method comprises:
   Step (a) culturing a microbial cell that comprises:
   i) at least one copy of the single strand of DNA of any of paragraphs 1-70 present on a phagemid, wherein the one or each copy of the single strand of DNA is flanked by a self-cleaving DNA sequence, to produce a precursor template ssDNA, wherein the phagemid comprises a selection marker, optionally an antibiotic resistance gene, and a packaging sequence; and
   ii) one or more phage genes, that encode for phage proteins (GenIII); and
   iii) genes encoding the proteins of a bacteriophage, optionally genes encoding the proteins of M13 bacteriophage, optionally wherein said genes are present on a helper plasmid
   under conditions so as to produce a population of cells and allow the cells to produce phage comprising the at least one copy of the single strand of DNA of any of paragraphs 1-70 that are released from the cells.
102. The method of paragraph 101 further comprising step (b) harvesting the phage from the culture media.
103. The method of paragraph 102 further comprising step (c) extracting the ssDNA from the phage and exposing said ssDNA to conditions so as to allow self-cleavage of the self-cleaving DNA sequence.
104. The method of paragraph 103 further comprising step (d) isolating the single strand of DNA of any of paragraphs 1-70.
105. The method of any of paragraphs paragraph 101-104 wherein steps (a); (a) and (b); (a), (b) and (c); or (a), (b), (c) and (d) are completed within a 48 hour, 72 hour, or 96 hour, 108 hour, 120 hour, 132 hour, 144 hour, 156 hour, 168 hour, 180 hour period.
106. The method of any of paragraphs 101-105 wherein:
   a) 50g, 75g, 100g, 125g, 150g, 175g, 200g of isolated single strand of DNA is produced; and/or
   b) 50mg/L, 75mg/L, 100mg/L, 125mg/L, 150mg/L, 175mg/L, 200mg/L of isolated single strand of DNA is produced.
107. The method of any of paragraphs 101-106 wherein:
   a) 50g, 75g, 100g, 125g, 150g, 175g, 200g of isolated single strand of DNA is produced; and/or
   b) 50mg/L, 75mg/L, 100mg/L, 125mg/L, 150mg/L, 175mg/L, 200mg/L of isolated single strand of DNA is produced
   in a 48 hour, 72 hour, or 96 hour, 108 hour, 120 hour, 132 hour, 144 hour, 156 hour, 168 hour, 180 hour period.
108. A method of producing a vaccine comprising one or more single strand of DNA s of any of paragraphs 1-70 wherein said method comprises producing a single strand of DNA of any of paragraphs 1-70 according to paragraph 100, and formulating the isolated single strand of DNA of step (f) as a vaccine.
109. A method of producing a LNP vaccine comprising one or more single strand of DNA s of any of paragraphs 1-70 wherein said method comprises producing a single strand of DNA of any of paragraphs 1-70 according to paragraph 100, and combining the isolated single strand of DNA of step (f) with lipids.

### Figures

### Examples

### Design of ssDNA plasmid vectors optimized for expression and immunogenicity

Various factors such as regulatory elements, nuclear localisation signals for nuclear delivery and transcription, linear and circular ssDNA, sense and missense expression will be tested to optimise the single strand of DNA of the invention.

Scalable production methods specifically for the production of the single strand of DNA of the invention will be developed, with upstream and downstream processing optimised.

### In vitro evaluation for target sequence production

The ability of various iterations of single strands of DNA of the invention will be tested for the ability to transcribe the target sequence, and produce protein or peptides, or non-coding RNAs. The ability of antigenic proteins or peptides produced in this way will be tested for the ability to stimulate immune cells.

### In vivo testing in mice and rabbits

Promising candidates will be rapidly advanced to in vivo testing in mice and rabbits to assess immunogenicity, safety, and protection against viral challenge.

### Scalable production of single strands of DNA suitable for therapeutic purposes

In parallel with the above, a scalable GMP manufacturing process will be developed for clinical trial material production.

### Protection against pathogen infection

The efficacy of the single strand of DNA of the invention in providing protection to various pathogens will be demonstrated by the use of established animal challenge models will build confidence in the platform.

### Delivery methods

Various delivery methods will be investigated to determine the optimal delivery method for a given context, including the testing of LNP encapsulation and Jet Injection.

### Case studies

*1 - Development of an ssDNA vaccine against a newly identified influenza strain with high pandemic potential (avian flu H5Nx), from antigen selection to preclinical immunogenicity and challenge studies in animal models.*
*2* - *Adaptation of the platform to target a novel coronavirus, demonstrating the speed and flexibility of the approach in responding to unexpected outbreaks.*
*3* - *Comparison of ssDNA vaccines to conventional plasmid DNA and mRNA vaccines in terms of immunogenicity, manufacturing timelines, and stability.*

### Equivalents

The foregoing embodiments, instances, and examples are applicable to any of the aspects of the present disclosure and should be construed as such.

While the present disclosure has been described in terms of various aspects, embodiments, and examples, it is understood that variations, improvements, and equivalents will occur to the person skilled in the art. Such variations, improvements, and equivalents are contemplated by the present disclosure and fall within the scope of the matter disclosed and claimed herein.

## Claims

1. A single strand of DNA that:
comprises at least a first target sequence operably linked to a first promoter sequence, wherein the first promoter sequence drives expression of the first target sequence to produce a first RNA molecule either:
i) directly from the single strand of DNA; or
ii) following replication of the single strand of DNA into the double strand form.

2. The single strand of DNA of claim 1 wherein the single strand of DNA is designed so as to allow expression of the first target sequence to produce a first RNA molecule in humans.

3. The single strand of DNA of claim 1 or 2 wherein the single strand of DNA comprises at least one region that is double stranded, wherein said double stranded region is generated via hybridisation between at least a first and second region of the single strand of DNA that are substantially complementary to each other, and wherein the hybridisation between a first and second region of the single stand of DNA enhances expression of the first RNA molecule.

4. The single strand of DNA of any of claims 1-4 further comprising:
a) a cell-type specific promoter; and/or
b) a first enhancer sequence, wherein the first enhancer sequence enhances expression of the first target sequence in a specific cell type.

5. The single strand of DNA of any of the preceding claims wherein the single strand of DNA comprises any one or more modifications that:
a) reduce immunogenicity and wherein the modification is methylation;
b) make the single strand of DNA act as an adjuvant, and wherein the modification is demethylation.

6. The single strand of DNA of any of the preceding claims wherein the single strand of DNA additionally comprises a second target sequence, or a third, fourth, fifth, sixth, seventh, eighth, ninth or tenth or more target sequence.

7. The single strand of DNA of claim 6 wherein the at least two target sequences present in the single strand of DNA are arranged in a tandem array and are operably linked to the same promoter so that the target sequences are transcribed into a single RNA molecule, and wherein the single RNA molecule comprises an internal ribosome entry site (IRES) that is present 5' to each target sequence, so that each target sequence is translated.

8. The single strand of DNA of any of the preceding claims wherein the first target sequence is an antisense strand that is able to be directly transcribed to produce the first RNA molecule; or is a sense strand that is able to be transcribed to produce the first RNA molecule only once the single strand of DNA is replicated to produce the antisense strand.

9. The single strand of DNA according to any of the preceding claims wherein the at least first target sequence encodes a first protein or peptide that is an antigenic protein or peptide or comprises an antigenic protein or peptide, and wherein the antigenic protein or peptide is selected from the group comprising or consisting of:
a) a viral antigen;
b) a cancer antigen;
c) an Autoimmune Disease Antigen;
d) a Neurological Disease Antigens;
e) a bacterial antigen;
f) a fungal antigen; and/or
g) a protist antigen.

10. The single strand of DNA according to claim 9 wherein the first protein or peptide that is an antigenic protein or peptide or comprises an antigen protein or peptide comprises one or more sequences to enhance presentation of the antigen protein or peptide or fragments thereof on MHC I and/or MHC II.

11. The single strand of DNA according to claim 10 wherein the one or more sequences to enhance presentation of the first protein or peptide fragment is:
a) a ubiquitin sequence;
b) an Endoplasmic Reticulum (ER) signal peptide, optionally the signal peptide from tissue plasminogen activator (tPA);
c) the Lysosome-Associated Membrane Protein 1 (LAMP-1); and/or
d) a chemokine ligand, optionally XCL1.

12. The single strand of DNA according to any of the preceding claims wherein at least one target sequence comprises a sequence that encodes an adjuvant protein or peptide.

13. The single strand of DNA according to any of the preceding claims wherein at least one target sequence encodes a protein or peptide, and wherein the protein or peptide is a therapeutic protein or peptide capable of replacing a defective or deficient protein associated with a genetic disorder.

14. The single strand of DNA according to any of the preceding claims wherein at least one target sequence is a non-coding RNA selected from the group comprising or consisting of:
a) a gRNA;
b) an RNA designed to have sequence complementarity to a host target RNA so as to cause RNAi when expressed in a host cell, optionally a miRNA; and/or
c) a IncRNA;
optionally
wherein the RNA is designed to have sequence complementarity to a host target RNA that is RNA transcribed from an oncogene, optionally wherein the oncogene is selected from the group comprising or consisting of KRAS, EGFR (Epidermal Growth Factor Receptor), CFTR (Cystic Fibrosis Transmembrane Conductance Regulator), DMD (Dystriophin).

15. The single strand of DNA according to any of the preceding claims wherein the single strand of DNA comprises at least a first target sequence that encodes a protein or peptide, and at least a second target sequence that encodes a gRNA and wherein the protein or peptide is a Cas protein; optionally wherein the gRNA is designed to have sequence complementarity to a viral genome or viral nucleic acid, optionally wherein the virus is selected from the group comprising or consisting of HBV and wherein the viral nucleic acid is cccDNA (Covalently Closed Circular DNA), or HIV Provirus.

16. The single strand of DNA according to any of the preceding claims wherein the single strand of DNA comprises 2, 3, 4, 5, 6, 7, 8, 9, 10 or more target sequences that each encodes, or encodes following replication of the single strand of DNA, an antigenic protein or peptide; and wherein at least two of the target sequences encode, or encodes following replication of the single strand of DNA, different antigenic proteins or peptides, optionally at least 3, 4, 5, 6, 7, 8, 9 or at least 10 of the target sequences encode, or encode following replication of the single strand of DNA, different antigenic proteins or peptides.

17. The single strand of DNA according to any of the preceding claims wherein the single strand of DNA:
i) is not a closed linear DNA;
ii) is not doggybone DNA (dbDNA);
iii) does not comprise a telomerase target sequence or a protelomerase target sequence; and/or
iv) does not comprise a hairpin loop at the 5', 3', or 5' and 3' termini.

18. The single strand of DNA according to any of the preceding claims, wherein the single strand of DNA is:
At least 5000, 5250, 5500, 5750, 6000, 6250, 6500, 6750, 7000, 7250, 7500, 7750, 8000, 8250, 8500, 8750, 9000, 9250, 9500, 9750, 10000, 10250, 10500, 10750, 11000, 11250, 11500, 11750, 12000, 12250, 12500, 12750, 13000, 13250, 13500, 13750, 14000, 14250, 14500, 14750, 15000, 15250, 15500, 15750, 16000, 16250, 16500, 16750, 17000, 17250, 17500, 17750, 18000, 18250, 18500, 18750, 19000, 19250, 19500, 19750, or at least 20000 nucleotides in length.

19. A lipid nanoparticle (LNP) comprising one or more of the single strands of DNA of any one of the preceding claims.

20. A vaccine comprising one or more single strands of DNA according to any of claims 1-18.

21. The single strand of DNA according to any of claims 1-18, the LNP according to claim 19 or the vaccine according to claim 20 for use in:
a) a method of treating or preventing an infection with a pathogen, optionally a viral, bacterial, fungal or protist pathogen in a subject;
b) a method of treating or preventing a disease in a subject.
c) a method of treating or preventing cancer in a subject,
optionally wherein the single strand of DNA, LNP or vaccine is formulated for Nanojet delivery.

22. A method of producing the single strand of DNA of any of claims 1-18, wherein said method comprises:
Step (a) culturing a microbial cell that comprises:
i) at least one copy of the single strand of DNA of any of claims 1-18 present on a phagemid, wherein the one or each copy of the single strand of DNA is flanked by a self-cleaving DNA sequence, to produce a precursor template ssDNA, wherein the phagemid comprises a packaging sequence;
ii) one or more phage genes, that encode for phage proteins (GenIII); and
iii) genes encoding the proteins of a bacteriophage, optionally genes encoding the proteins of M13 bacteriophage, optionally wherein said genes are present on a helper plasmid;
and optionally comprises a selection marker, optionally an antibiotic resistance marker;
wherein said culturing is under conditions so as to produce a population of cells and allow the cells to produce phage comprising the at least one copy of the single strand of DNA of any of claims 1-18 that are released from the cells;
step (b) harvesting the phage from the culture media;
step (c) extracting the ssDNA from the phage and exposing said ssDNA to conditions so as to allow self-cleavage of the self-cleaving DNA sequence; and
step (d) isolating the single strand of DNA of any of claims 1-18;
and wherein steps (a), (b), (c) and (d) are completed within a 48 hour, 72 hour, or 96 hour, 108 hour, 120 hour, 132 hour, 144 hour, 156 hour, 168 hour, 180 hour period and produce:
i) 50g, 75g, 100g, 125g, 150g, 175g, 200g of isolated single strand of DNA of any of claims 1-18; and/or
ii) 50mg/L, 75mg/L, 100mg/L, 125mg/L, 150mg/L, 175mg/L, 200mg/L of isolated single strand of DNA of any one of claims 1-18.

23. A method of producing nucleic acid for use in a vaccine wherein the method produces:
i) 50g, 75g, 100g, 125g, 150g, 175g, 200g of isolated single strands of DNA; and/or
ii) 50mg/L, 75mg/L, 100mg/L, 125mg/L, 150mg/L, 175mg/L, 200mg/L of isolated single strands of DNA;
in a time period of 48 hours, 72 hours, or 96 hours, 108 hours, 120 hours, 132 hours, 144 hours, 156 hours, 168 hours, or 180 hours,
and wherein the method comprises the method of claim 22.
